(19) Europäisches Patentamt
European Patent Office
Office européen des brevets

(11) Veröffentlichungsnummer: **0 129 509**
**B1**

(12) # EUROPÄISCHE PATENTSCHRIFT

(45) Veröffentlichungstag der Patentschrift: **23.08.89**

(21) Anmeldenummer: **84810264.6**

(22) Anmeldetag: **29.05.84**

(51) Int. Cl.[4]: **C 07 D 487/04,** A 61 K 31/55 // C07D249/08, C07D249/10, C07D249/12, C07D403/12, (C07D487/04, 249:00, 243:00)

(54) **Triazol(2,3-c)(1,3)benzodiazepine, Verfahren zu ihrer Herstellung, pharmazeutische Präparate enthaltend diese Verbindungen, sowie ihre therapeutische Verwendung.**

(30) Priorität: **06.06.83 US 501328**

(43) Veröffentlichungstag der Anmeldung: **27.12.84 Patentblatt 84/52**

(45) Bekanntmachung des Hinweises auf die Patenterteilung: **23.08.89 Patentblatt 89/34**

(84) Benannte Vertragsstaaten: **AT BE CH DE FR GB IT LI LU NL SE**

(56) Entgegenhaltungen:
**EP-A-0 081 461**
**US-A-4 076 823**
**US-A-4 192 803**

(73) Patentinhaber: **CIBA- GEIGY AG, Klybeckstrasse 141, CH- 4002 Basel (CH)**

(72) Erfinder: **Vlattas, Isidoros, Dr., 131 Butler Parkway, Summit New Jersey 07901 (US)**

**EP 0 129 509 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

In der U.S. Patentschrift 4 076 823 sind 5H-[1,2,4]triazolo-[5,1-a][2,4]benzodiazepine mit antiinflammatorischen, sedativen, anxiolytischen Eigenschaften sowie Eigenschaften als Muskelrelaxantien beschrieben.

In der U.S. Patentschrift 4 192 803 sind 5H-Pyrrolo[2,1-c][1,4]-benzodiazepine beschrieben. Diese Verbindungen unterscheiden sich von den aus der U.S. Patentschrift 4 076 823 bekannten Verbindungen durch Anellierung des Triazol-Anellanden an einer anderen Stelle des Benzodiazepingerüsts sowie durch unterschiedliche Anordnung der Stickstoffatome im Benzodiazepingerüst selbst. Wirkungsmäßig unterscheiden sie sich durch ihre antipsychotischen und antidepressiven Eigenschaften.

Die nachveröffentlichte Europäische Patentschrift 81 461 hat 11H-Imidazol[1,2-c][1,3]benzodiazepine zum Gegenstand, welche sich um ein Stickstoffatom von den 11H-1,2,4-triazol[2,3-c][1,3]benzol-diazepinen der vorliegenden Erfindung unterscheiden.

Die Erfindung betrifft 5-Diazacycloalkyl-1,2,4-triazol[2,3-c]-[1,3]benzodiazepine der Formel I

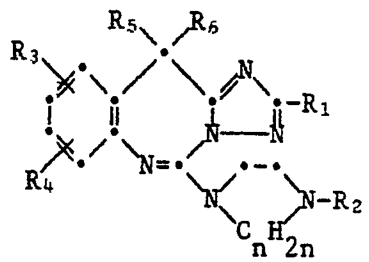

(I),

worin $R_1$ Wasserstoff, $C_1$-$C_4$-Alkylthio, Amino, ($C_1$-$C_4$-Alkanoyl-, $C_1$-$C_4$-Alkoxycarbonyl-, Carbamoyl-, Sulfamoyl-, $C_1$-$C_4$-Monoalkyl-, oder Di-$C_1$-$C_4$-alkylcarbamoyl- oder -sulfamoyl-, Halosulfonyl-, Phenyl-$C_1$-$C_2$-alkoxycarbonyl-, $C_1$-$C_4$-Alkyl- oder Di-$C_1$-$C_4$-alkyl)-amino, $C_1$-$C_4$-Alkoxy, ($C_1$-$C_4$-Alkanoyl-, $C_1$-$C_4$-Alkoxycarbonyl-, Carbamoyl-, Sulfamoyl-, mono-$C_1$-$C_4$-Alkyl- oder di-$C_1$-$C_4$-Alkylcarbamoyl- oder -sulfamoyl-, Halosulfonyl- oder Phenyl-$C_1$-$C_2$-alkoxycarbonyl-)oxy, $C_1$-$C_4$-Alkyl, Acetyl, Propionyl, Hydroxy, Halogen, Trifluormethyl, Cyano, Carboxy, Methoxy- oder Äthoxycarbonyl, Carbamoyl, Mono- oder Dimethyl- oder -äthylcarbamoyl, Hydroxy-$C_1$-$C_4$-alkyl oder Di-$C_1$-$C_4$-alkylamino-$C_1$-$C_4$-alkyl bedeutet;

$C_nH_{2n}$ für $C_2$-$C_4$-Alkylen steht, welches die zwei Stickstoffatome durch 2 oder 3 Kohlenstoffatome trennt;

$R_2$ für Wasserstoff, $C_1$-$C_7$-Alkyl, Allyl, Propargyl, Acetyl, Propionyl, Phenyl-$C_1$-$C_3$-alkyl, welches am Phenylring durch Halogen, $C_1$-$C_4$-Alkoxy oder $C_1$-$C_4$-Alkyl substituiert sein kann, Methoxy- oder Äthoxycarbonyl, Phenylmethoxycarbonyl, Phenyläthoxycarbonyl, 2-Hydroxy-(äthyl oder propyl), 3-Hydroxy-(propyl oder butyl), 4-Hydroxybutyl, $C_2$-$C_4$-Alkanoyloxy-$C_2$-$C_4$-alkyl, Phenoxy-$C_2$-$C_4$-alkyl, welches am Phenylring durch Halogen, $C_1$-$C_4$-Alkoxy oder $C_1$-$C_4$-Alkyl substituiert sein kann, oder $C_1$-$C_4$-Alkoxy-$C_2$-$C_4$-alkyl;

$R_3$ und $R_4$ unabhängig voneinander Wasserstoff, $C_1$-$C_7$-Alkyl, $C_1$-$C_4$-Alkoxy, $C_1$-$C_4$-Alkylthio, Halogen, Trifluormethyl, Hydroxy, Acetoxy, Propionyloxy, Sulfamoyl, Mono- oder Di-$C_1$-$C_4$-alkylsulfamoyl und $R_5$ und $R_6$ Wasserstoff oder $C_1$-$C_4$-Alkyl bedeuten; ihre N-Oxide, und Salze.

Die allgemeinen Definitionen wie sie hier gebraucht werden, haben im Rahmen der vorliegenden Erfindung die unten beschriebene Bedeutung.

Ein Halogenatom ist vorzugsweise Fluor oder Chlor, aber auch Brom oder Jod.

Eine $C_1$-$C_4$-Alkylgruppe oder eine solche in den genannten $C_1$-$C_4$-Alkoxy-, $C_1$-$C_4$-Alkylthio- oder anderen genannten alkylierten Gruppen ist vor allem Methyl und auch Äthyl, n- oder iso-(Propyl oder Butyl).

Eine $C_1$-$C_7$-Alkylgruppe ist ferner n- oder iso-(Pentyl, Hexyl oder Heptyl), z. B. 2-Methylpropyl oder 3-Methylbutyl.

Phenyl-$C_1$-$C_3$-alkyl bedeutet Benzyl, 1-, 2- oder 3-Phenylpropyl oder 1- oder 2-Phenyläthyl, welches am Phenylring gegebenenfalls durch Halogen, $C_1$-$C_4$-Alkoxy oder $C_1$-$C_4$-Alkyl substituiert sein kann.

Eine $C_1$-$C_4$-Alkoxygruppe bedeutet beispielsweise Äthoxy, Propoxy, Isopropoxy oder insbesondere Methoxy.

Eine $C_1$-$C_4$-Alkylthiogruppe bedeutet beispielsweise Äthylthio, Propylthio oder insbesondere Methylthio.

$C_1$-$C_4$-Alkanoyl steht vorzugsweise für Acetyl oder Propionyl. $C_1$-$C_4$-Alkanoyloxy steht vorzugsweise für Acetoxy oder Propionyloxy.

Eine $C_1$-$C_4$-Alkoxycarbonyl-, Mono- oder Di-$C_1$-$C_4$-alkylcarbamoyl- oder Mono- oder Di-$C_1$-$C_4$-alkylsulfamoylgruppe ist vorzugsweise Methoxycarbonyl, Äthoxycarbonyl; Mono- oder Dimethylcarbamoyl bzw. Mono- oder Dimethylsulfamoyl.

Eine Phenyl-$C_1$-$C_4$-alkoxycarbonylgruppe ist z. B. Phenylmethoxycarbonyl oder Phenyläthoxycarbonyl.

Die $C_2$-$C_4$-Alkylengruppe $C_nH_2$ ist insbesondere Äthylen, aber auch 1,2- oder 1,3-Propylen, 1,2-, 1,3- oder 2,3-Butylen, welche mit den benachbarten Stickstoffatomen vorzugsweise eine Piperazino- oder Homopiperazinogruppe bilden.

Eine $C_2$-$C_4$-Alkanoyloxy-$C_2$-$C_4$-alkylgruppe bedeutet vorzugsweise $C_2$-$C_4$-Alkanoyloxy-(äthyl, propyl oder butyl), beispielsweise 2-Acetoxy- oder 2-Propionyloxy-(äthyl, propyl oder butyl), 3-Acetoxy- oder 3-Propionyloxy-(propyl oder butyl), 4-Acetoxy- oder 4-Propionyloxy-butyl.

Eine $C_1$-$C_4$-Alkoxy-$C_2$-$C_4$-alkylgruppe bedeutet vorzugsweise $C_1$-$C_4$-Alkoxy-(äthyl, propyl oder butyl), wie z. B.

2-Methoxy- oder Äthoxy-(äthyl, propyl oder butyl), 3-Methoxy- oder 3-Äthoxy(propyl oder butyl), 4-Methoxy- oder 4-Äthoxybutyl.

Eine Phenoxy-$C_2$-$C_4$-alkylgruppe bedeutet vorzugsweise Phenoxy(äthyl, propyl oder butyl), wobei solche Gruppen gegebenenfalls im Phenylring vorzugsweise durch Halogen, $C_1$-$C_4$-Alkoxy oder auch $C_1$-$C_4$-Alkyl substituiert sein können.

Die Verbindungen der Formel I bilden Säureadditionssalze, vorzugsweise mit therapeutisch verwendbaren anorganischen oder auch organischen Säuren, wie z. B. mit starken Mineralsäuren, wie Halogenwasserstoffsäuren, z. B. Chlorwasserstoff- oder Bromwasserstoffsäure, Schwefel-, Phosphor- oder auch Salpetersäure oder mit organischen Säuren, wie z. B. aliphatischen oder aromatischen Carbon- oder Sulfonsäuren, z. B. Ameisen-, Essig-, Propion-, Bernstein-, Glycol-, Milch-, Äpfel-, Wein-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoa-, Nicotin-, Methansulfon-, Äthansulfon-, Hydroxyäthansulfon-, Äthylensulfon-, Halogenbenzolsulfon-, Toluolsulfon-, Naphthalinsulfon-, Sulfanil- oder Cyclohexylsulfaminsäure; oder die Ascorbinsäure.

Gewisse Verbindungen der Formel I, in welchen $R_1$ Carboxy bedeutet, bilden auch Salze mit Basen, vorzugsweise solchen, welche therapeutisch verwendbare Salze ergeben, z. B. Ammoniak, Mono-, Di- oder Triniederalkylamine, Niederalkylenamine, Morpholin, Piperazin, Piperidin oder mit Niederalkylderivaten der genannten cyclischen Basen; Alkalimetall- oder Erdalkalimetallhydroxyde.

Die Verbindungen der Erfindung zeigen wertvolle pharmakologische Eigenschaften, insbesondere psychoaktive, wie z. B. neuroleptische Wirkungen, aber auch antiallergische, wie z. B. antihistaminische Wirkungen. Diese können in Tierversuchen, vorzugsweise an Säugern, z. B. Mäusen, Ratten, Meerschweinchen oder Affen, als Testobjekte, nachgewiesen werden. Die genannten Verbindungen können ihnen enteral oder parenteral, vorzugsweise oral oder subcutan, intravenös oder intraperitoneal, z. B. durch Steckkapseln oder in Form von Stärke enthaltenden Suspensionen bzw. wässrigen Lösungen, verabreicht werden. Die verwendete Dosis kann in einem Bereich von ungefähr zwischen 0,1 und 50 mg/kg/Tag, vorzugsweise ungefähr 0,3 und 30 mg/kg/Tag, insbesondere zwischen 1 und 20 mg/kg/Tag, liegen.

Die genannten neuroleptischen Eigenschaften können an ausgewachsenen Ratten oder Totenkopfäffchen (squirrel monkeys) nachgewiesen werden. Die Tiere sind zur Betätigung eines Hebels trainiert, wodurch sie einem auf den Fuß verabreichten elektrischen Schock ausweichen können. Jeder Hebeldruck verschiebt den Schock um 30 Sekunden. Vergißt das Tier den Hebel innerhalb des genannten Zeitintervals einmal zu drücken, so werden alle 15 Sekunden kurze (0,5 Sekunden) elektrische Schocks abgegeben, bis das Tier den Hebel wieder betätigt. Unter Kontrollbedingungen drücken die Versuchstiere den Hebel mit einer mäßig ausgeglichenen Geschwindigkeit und erhalten selten mehr als 5 oder 6 Schocks während einer 25-minütigen (Ratten) bis 4-stündigen Versuchsperiode. Die genannten Verbindungen, welche den Tieren 30, 90 und 210 Minuten vor dem Versuch verabreicht werden, blockieren das angelernte konditionierte Vermeidungs-Verhalten. Die Folge ist die Abnahme der Vermeidungs-Reaktion und eine wesentliche Zunahme der von den Tieren erlittenen Schocks. Sowohl die Anzahl der Vermeidungs-Reaktionen als auch diejenige der Fehlverhalten (der erhaltenen Schocks) werden zur Auswertung registriert.

Schließlich können die antihistaminischen Eigenschaften in vitro gemäß Chasin et al., J. Neurochem. 22, 1031 (1974) nachgewiesen werden. Homogenate eines zellfreien Präparates der Grosshirnrinde von Meerschweinchen werden vorher mit $^3H$-Adenin inkubiert, wobei endogenes $^3H$-Adenosin-triphosphat gebildet wird. Die Homogenate werden dann mit 50 mikromolarem Histamin inkubiert, um die Synthese von $^3H$-cyclischem Adenosinmonophosphat zu aktivieren. Dieses Vorgehen wird in Gegenwart oder Abwesenheit der Testverbindung unternommen, wobei man Konzentrationen zwischen 0,01 und 100 Mikromol einsetzt. Ist die geprüfte Substanz aktiv, so wird die Histaminaktivierung der Adenylat-Cyclase gehemmt. Der $IC_{50}$-Wert bedeutet die Konzentration, bei welcher die Histamin-Aktivierung um 50 % gehemmt wird.

Die erfindungsgemäßen Verbindungen können dementsprechend als Neuroleptikum und als Antihistaminpräparat, z. B. in der Behandlung und Handhabung von psychotischen Erscheinungen, wie z. B. Agressions-, Agitations-, Schizophrenieerscheinungen und/oder von allergischen Zuständen von Säugern, inklusive Menschen, verwendet werden. Sie können auch als Zwischenprodukt zur Herstellung von anderen wertvollen Produkten, insbesondere von pharmakologisch wirksamen Präparaten, eingesetzt werden.

Bevorzugte erfindungsgemäße Verbindungen sind diejenigen der Formel I, worin $R_1$ Wasserstoff, $C_1$-$C_4$-Alkyl, Halogen, Trifluormethyl, $C_1$-$C_4$-Alkoxy oder $C_1$-$C_4$-Alkylthio bedeutet; und $C_nH_{2n}$ für $C_2$-$C_4$-Alkylen steht, welches die zwei Stickstoffatome durch 2 oder 3 C-Atome trennt, $R_2$ für Wasserstoff, $C_1$-$C_4$-Alkyl, Methoxycarbonyl, Äthoxycarbonyl oder Hydroxy-$C_2$-$C_4$-alkyl steht, $R_3$ Wasserstoff, $C_1$-$C_4$-Alkyl, $C_1$-$C_4$-Alkoxy, $C_1$-$C_4$-Alkylthio, Halogen oder Trifluormethyl bedeutet, und $R_4$ für Wasserstoff steht; und $R_5$ und $R_6$ die Bedeutung von Wasserstoff oder $C_1$-$C_4$-Alkyl haben, ihre N-Oxide und Salze.

Bevorzugt sind weiter Verbindungen der Formel I, worin $R_1$ Wasserstoff, Methyl, Äthyl, Methylthio, Chlor, Methoxy oder Trifluormethyl bedeutet; n für die ganze Zahl 2 oder 3 steht; $R_2$ für Wasserstoff, $C_1$-$C_4$-Alkyl, Methoxycarbonyl, Äthoxycarbonyl, Hydroxyäthyl oder Hydroxypropyl steht; $R_3$ Wasserstoff, Methyl, Methoxy, Methylthio, Chlor oder Trifluormethyl; $R_4$ Wasserstoff und $R_5$ und $R_6$ Wasserstoff oder Methyl bedeuten; ihre N-Oxide und Salze.

Besonders hervorzuheben sind Verbindungen der Formel II

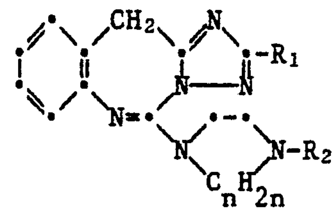

(II),

worin $R_1$ Wasserstoff, Halogen, Trifluormethyl, $C_1$-$C_4$-Alkylmercapto, $C_1$-$C_4$-Alkoxy, oder $C_1$-$C_4$-Alkyl bedeutet; $R_2$ für Wasserstoff, $C_1$-$C_4$-Alkyl oder Hydroxy-$C_2$-$C_4$-alkyl steht, worin Hydroxy vom Stickstoffatom durch mindestens 2 Kohlenstoffatome getrennt ist; $R_3$ Wasserstoff, $C_1$-$C_4$-Alkyl, $C_1$-$C_4$-Alkoxy, $C_1$-$C_4$-Alkylthio, Halogen oder Trifluormethyl bedeutet; $C_nH_{2n}$ für Äthylen oder Propylen steht; ihre N-Oxide und Salze.

Von besonderem Interesse sind diejenigen Verbindungen der Formel II, worin $R_1$ Wasserstoff, Halogen, $C_1$-$C_4$-Alkylthio, $C_1$-$C_4$-Alkoxy oder $C_1$-$C_4$-Alkyl; $R_2$ Wasserstoff, $C_1$-$C_4$-Alkyl oder Hydroxy-$C_2$-$C_4$-alkyl bedeuten, worin Hydroxy vom Stickstoffatom durch 2 oder 3 Kohlenstoffatome getrennt ist; $R_3$ für Wasserstoff, $C_1$-$C_4$-Alkyl, Halogen oder Trifluormethyl steht; $C_nH_{2n}$ Äthylen bedeutet, ihre N-Oxide und therapeutisch verwendbaren Salze davon.

Desweiteren von besonderer Bedeutung sind Verbindungen der Formel II, worin $R_1$ Wasserstoff, Methyl, Äthyl, Chlor, Methylthio oder Methoxy; $R_2$ Wasserstoff, Methyl, Äthyl, Propyl, 2-Hydroxyäthyl oder 3-Hydroxypropyl; $R_3$ Wasserstoff, Methyl, Methoxy, Fluor, Chlor oder Trifluormethyl und $C_nH_{2n}$ Äthylen bedeutet, und ihre therapeutisch verwendbaren Salze.

Besonders als Vertreter der erfindungsgemäßen Verbindungen weist die Verbindung des nachfolgenden Beispiels 1, nämlich das 2-Methyl-5-(4-methyl-1-piperazinyl)-11H-1,2,4-triazol[2,3-c][1,3]benzodiazepinmaleat, auf die antipsychotischen Eigenschaften hin. Die Verbindung vermindert die Vermeidungs-Reaktionen (Zunahme der Fehlverhalten) bei Ratten und Totenkopfäffchen bei einer oral verabreichten Gesamtdosis von ungefähr 1,0 mg/kg oder darunter.

Ferner sei noch zu erwähnen, daß die Verbindung des nachfolgenden Beispiels 1 bei wirksamen antipsychotischen Dosen sich auch dadurch auszeichnet, daß sie im wesentlichen frei von extrapyramidalen Nebenwirkungen ist, wie z. B. Diskinesien und Distonien im Totenkopfäffchen.

Die antihistaminische Wirkung wird insbesondere von 2-Methyl-5-(4-methyl-1-piperazinyl)-11H-1,2,4-triazolo[2,3c][1,3]benzodiazepinmaleat (Verbindung von Beispiel 11) veranschaulicht. Diese Verbindung hemmt die Histaminaktivierung der Adenylat-Cyclase mit einem $IC_{50}$ von ungefähr $3 \times 10^{-6}$ M.

Die Verbindungen der Erfindung werden nach an sich bekannten Methoden, z. B. dadurch hergestellt, daß man

a) eine Verbindung der Formel III

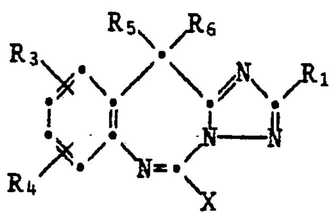

(III),

worin X eine zusammen mit Wasserstoff oder einem Alkalimetall abspaltbare Gruppe bedeutet und die anderen Symbole die unter der Formel I angegebenen Bedeutungen haben, mit einer Verbindung der Formel IV

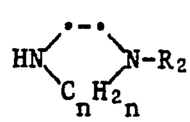

(IV)

oder einem Alkalimetallderivat davon, in welcher $R_2$ die unter der Formel I angegebene Bedeutung hat, kondensiert, und, wenn erwünscht, eine erhaltene Verbindung der Formel I in eine andere Verbindung der Erfindung umwandelt und, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomere oder Razemate auftrennt, und/oder, wenn erwünscht, erhaltene Razemate in die optischen Antipoden aufspaltet.

Eine zusammen mit Wasserstoff oder einem Alkalimetallatom abspaltbare Gruppe ist z. B. in erster Linie eine

freie oder vorzugsweise verätherte Mercaptogruppe, ferner eine gegebenenfalls funktionell abgewandelte reaktionsfähige Hydroxygruppe, die Cyanato- oder Thiocyanatogruppe oder die Nitroaminogruppe. Eine verätherte Mercaptogruppe ist in erster Linie eine durch einen gegebenenfalls substituierten Kohlenwasserstoffrest, insbesondere aliphatischen Charakters, verätherte Mercaptogruppe. Sie stellt in erster Linie Niederalkylthio, z. B. Methylthio, Äthylthio oder Butylthio oder Phenylniederalkylthio, z. B. Phenylthio oder Benzylthio dar. Eine gegebenenfalls funktionell abgewandelte reaktionsfähige Hydroxygruppe ist eine freie und unter anderem eine veresterte Hydroxygruppe. Eine solche ist unter anderem Halogen, z. B. Chlor oder Brom, oder Niederalkylsulfonyloxy, z. B. Methansulfonyloxy. Eine verätherte Hydroxygruppe ist beispielsweise eine Niederalkoxygruppe, wie z. B. Methoxy oder Äthoxy.

Diese Kondensation erfolgt vorteilhafterweise in einem Überschuß der eingesetzten Verbindung der Formel IV oder mit einer äquivalenten Menge der in situ hergestellten Alkalimetallverbindung davon, wenn X als abspaltbare Gruppe einer Verbindung der Formel III vorzugsweise Halogen, Niederalkylthio oder Thiocyanato bedeutet. Die Umsetzung erfolgt je nach der Bedeutung von X bei Temperaturen zwischen 0° und 150°C, vorzugsweise in einem geeigneten Lösungsmittel, wie z. B. Niederalkanol, beispielsweise Amylalkohol, Dimethylformamid, Hexamethylenphosphoramid oder Toluol. Die besagte Kondensation einer Verbindung der Formel III mit einer Verbindung der Formel IV kann jedoch auch in Gegenwart einer Säure, beispielsweise einer Halogenwasserstoffsäure wie z. B. Chlorwasserstoff (Salzsäure) ausgeführt werden.

Die neuen 11H-1,2,4-Triazol[2,3c][1,3]benzodiazepin-Ausgangsstoffe der Formel III werden nach an sich bekannten Ringschluss-Methoden hergestellt. Vorteilhafterweise kondensiert man Verbindungen der Formel V

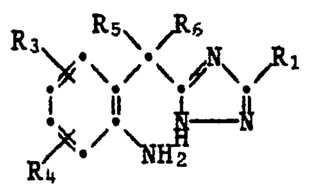

(V),

worin $R_1$ und $R_3$ bis $R_6$ die unter der Formel I angegebenen Bedeutungen haben, mit reaktionsfähigen Kohlensäurederivaten, wie z. B. Phosgen, Thiophosgen, 1,1-Carbonyldiimidazol, Bromcyan oder Chlorameisensäurephenylester.

Verbindungen der Formel III, in welchen X Hydroxy bedeutet, können in solche, worin X für Sulfhydryl steht, mit konventionellen Sulfurierungsmitteln, z. B. mit Phosphorpentasulfid umgewandelt werden.

Verbindungen der Formel III, d.h. worin X Hydroxy oder Sulfhydryl bedeutet, können weiter derivatisiert werden, zu Verbindungen der Formel III, worin X die oben angegebene Bedeutung hat, und zwar nach an sich bekannten Methoden oder nach Verfahren analog zu denen wie sie in den Beispielen beschrieben sind.

Ausgangsverbindungen der Formel V werden vorzugsweise durch Reduktion der entsprechend verschieden substituierten 5-(o-Nitrobenzyl)-1,2,4-triazolen durch Reduktion hergestellt, die wieder vorzugsweise aus den entsprechend substituierten o-Nitrobenzylnitrilen und daraus abgeleiteten Niederalkyliminoethern mit den Hydraziden der Formel $R_1$-$CONHNH_2$, worin $R_1$ die oben für die Verbindungen der Formel I angegebene Bedeutung hat, nach bekannten, analog zu den in den Beispielen beschriebenen Verfahren hergestellt werden.

Ein weiteres Verfahren zur Herstellung von Verbindungen der allgemeinen Formel I, besteht darin, dass man b) eine Verbindung der Formel VI

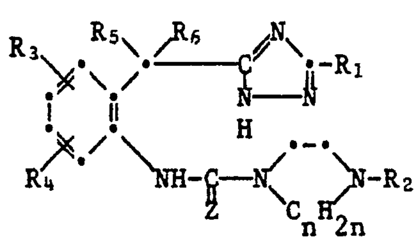

(VI),

worin Z ein Sauerstoff- oder Schwefelatom oder NH bedeutet, unter entwässernden, dehydrosulfurierenden oder desaminierenden Bedingungen ringschliesst, und die anderen Symbole die oben angegebenen Bedeutungen haben, und, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomere oder Razemate auftrennt, und/oder, wenn erwünscht, erhaltene Razemate in die optischen Antipoden aufspaltet.

Der genannte Ringschluß wird vorzugsweise bei einer Temperatur wischen 0° und 120°, mit einem

Reaktionsmittel wie Phosphorhalogeniden und/oder -oxyhalogeniden, wie z. B. Phosphorpentachlorid oder Phosphoroxychlorid oder Cyanhalogeniden, wie Bromcyan, mit oder ohne Kronenätherkatalysator, z. B. 18-Kronen-6-äther, in Gegenwart oder Abwesenheit von basischen Katalysatoren, wie z. B. Triäthylamin oder Kaliumcarbonat, vorzugsweise in einem inerten Lösungsmittel, z. B. Acetonitril oder Toluol durchgeführt.

Die Ausgangsstoffe der Formel VI können, falls sie neu sind, nach an sich bekannten Methoden erhalten werden. Beispielsweise können die Ausgangsstoffe der Formel VI ausgehend von den (tautomeren) Vorstufen-Verbindungen der Formel III, worin X Hydroxy, Thio oder Amino bedeutet, hergestellt werden. Diese Verbindungen werden mit Verbindungen der Formel IV in Gegenwart oder Abwesenheit von anderen Basen, z. B. den obengenannten, vorzugsweise in inerten Lösungsmitteln, wie Methylenchlorid oder Toluol, bei Temperaturen zwischen 0° und 150°C, vorteilhafterweise zwischen 10° und 50° kondensiert. Die Ringöffnung wird vorzugsweise bei niedrigen Temperaturen durchgeführt, um Nebenreaktionen der gegebenenfalls vorhandenen reaktionsfähigen funktionellen Gruppen $R_1$ bis $R_4$ vorzubeugen.

Auf einem anderen Wege können Ausgangsstoffe der Formel VI, in welchen $R_2$ Niederalkanoyl, Niederalkoxycarbonyl oder Phenyl-niederalkoxycarbonyl bedeutet, durch Kondensation von Verbindungen der Formel V mit einer Verbindung der Formel VII

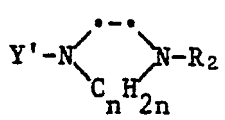 (VII),

worin Y'-Halogencarbonyl, Halogenthiocarbonyl oder Cyan bedeuten, vorzugsweise in einem inerten Lösungsmittel wie bereits oben angegeben, bei Temperaturen zwischen 0° und 150°C in An- oder Abwesenheit eines basischen Katalysators, wie z. B. Triäthylamin oder Kaliumcarbonat hergestellt werden.

Ausgangsverbindungen der Formel VII werden vorzugsweise aus Verbindungen der Formel IV, worin $R_2$ die unter der Formel VII angegebenen Bedeutungen hat, oder aus den entsprechenden N-Trimethylsilylderivaten durch Umsetzung mit Phosgen, Thiophosgen oder Bromcyan in einem inerten Lösungsmittel, wie z. B. Diäthyläther, Methylenchlorid oder Dimethoxyäthan bei Temperaturen zwischen -70°C und +50°C erhalten. Die Umsetzungen können in An- oder auch Abwesenheit eines basischen Katalysators, beispielsweise Triäthylamin oder Kaliumcarbonat ausgeführt werden.

Die erhaltenen Verbindungen können in an sich bekannter Weise in andere Verbindungen der Formel I übergeführt werden. So können z. B. Verbindungen, in welchen $R_2$ Wasserstoff oder ein Alkalimetallatom bedeutet, z. B. ihre Natrium- oder Lithiumsalze, mit substituierten oder unsubstituierten Oxiranen, z. B. mit Äthylenoxyd, oder mit reaktionsfähigen Estern von unsubstituierten oder entsprechend substituierten aliphatischen oder araliphatischen Alkoholen, z. B. Methanol, Äthanol, Methoxyäthanol, Phenoxyäthanol, Allyl- oder Propargylalkohol, in die entsprechenden N-substituierten Verbindungen, je nach der molaren Menge des verwendeten Alkylierungsmittels, übergeführt werden. Diese Ester sind beispielsweise von starken anorganischen oder organischen Säuren, vor allem von Halogenwasserstoffsäuren, z. B. Chlorwasserstoff-, Bromwasserstoff- oder Jodwasserstoffsäure, Schwefelsäure oder einer aromatischen Sulfonsäure, z. B. p-Toluolsulfonsäure oder m-Brombenzolsulfonsäure abgeleitet. Zwischenprodukte der Formel I in welchen $R_2$ ein Alkalimetallatom bedeutet oder auch Alkalimetallderivate von Verbindungen der Formel IV können durch Metallisierung mit reaktionsfähigen organischen Metallverbindungen, z. B. Lithiumdiisopropylamid, mit Alkalimetallalkoxyden, z. B. Natriummethoxid (Natriummethylat) oder mit Alkalimetallhydriden, z. B. Natrium- oder Kaliumhydrid, erhalten werden.

Ungesättigte Verbindungen z. B. solche, in welchen $R_2$ Allyl oder Propargyl bedeutet, können mit katalytisch aktiviertem Wasserstoff in die Verbindungen, in welchen $R_2$ den entsprechenden Propylrest bedeutet, umgewandelt werden. Umgekehrt können erhaltene N-alkylierte Verbindungen in N-unsubstituierte Verbindungen umgewandelt werden. Man hydrogenolysiert katalytisch die N-Benzylverbindungen oder man setzt sie z. B. mit Halogenameisensäure-niederalkylestern, z. B. Chlorameisensäure-äthylester um, wobei man N-Acylderivate erhält, welche z. B. mit wässrigen Basen, z. B. Alkalimetallhydroxiden, wie eine wässrige Natriumhydroxidlösung in die genannten N-unsubstituierten Verbindungen ($R_2$ = H) übergeführt werden können.

Verbindungen der Formel I, in welchem $R_2$ Hydroxy-$C_2$-$C_4$-alkyl bedeutet, können auch dadurch hergestellt werden, daß man entsprechende Verbindungen der Formel I, in welchen $R_2$ Wasserstoff bedeutet, zuerst mit reaktionsfähigen Derivaten von entsprechenden Glykolen, Glykolsäuren oder Dicarbonsäuren, z. B. ihren Niederalkylestern, Halogeniden oder Anhydriden, oder mit reaktionsfähigen Estern der genannten Glykole oder Glykolsäurederivate, z. B. mit Estern von Halogenwasserstoffsäuren oder aromatischen Sulfonsäuren, 1,2-Dibromäthan oder 1,2-Dibrompropan, Bromessigsäure-äthylester oder Brompropionsäure-äthylester, Tosyloxyessigsäure-äthylester, Oxalsäure-diäthylester, Malonsäure-diäthylester oder Oxalsäure-monoäthylesterchlorid umsetzt. Die erhaltenen Zwischenprodukte werden entweder hydrolysiert oder mit einfachen oder komplexen Leichtmetallhydriden, z. B. Lithiumaluminiumhydrid, allein oder mit Diboran, zu den Verbindungen der Formel I, in welchen $R_2$ Hydroxy-$C_2$-$C_4$-alkyl bedeutet, reduziert.

Verbindungen der Formel I, in welchen $R_2$ $C_1$-$C_4$-Alkyl, beispielsweise Methyl, bedeutet, können wie folgt hergestellt werden: Anfangs werden Verbindungen, in welchen $R_2$ für Wasserstoff steht, durch Umsetzung mit Halogenameisensäure-niederalkylestern oder -phenylniederalkylestern, z. B. Chlorameisensäure-äthylester, in

die Verbindungen der Formel I, worin $R_2$ Methoxy- oder Äthoxycarbonyl oder Phenylmethoxycarbonyl bedeutet, übergeführt. Dann reduziert man diese Acylderivate mit einfachen oder komplexen Leichtmetallhydriden, z. B. mit Lithiumaluminiumhydrid, Natrium-tri-tert.butoxy-aluminium-hydrid oder Natrium-bis-(2-methoxy-äthoxy)-aluminium-hydrid.

N-Acylderivate, in welchen $R_2$ beispielsweise eine Acetyl- oder Propionylgruppe bedeutet, können aus Verbindungen der Formel I, in welchem $R_2$ Wasserstoff bedeutet und entsprechenden reaktionsfähigen Säurederivaten, z. B. Säurehalogeniden, einfachen oder aktivierten Estern, z. B. Alkylestern oder Cyanalkylestern, Anhydriden oder Isocyanaten erhalten werden. Diese erhaltenen Verbindungen können wiederum in Verbindungen der Formel I, worin $R_2$ Niederalkyl bedeutet, wie oben angegeben reduziert werden. Verbindungen der Formel I, in welcher $R_2$ Hydroxy-$C_2$-$C_4$-alkyl bedeutet, können wie oben angegeben zu Verbindungen, in denen $R_2$ $C_2$-$C_4$-Alkanoyloxy-$C_2$-$C_4$-alkyl bedeutet, acyliert werden.

Erhaltene Verbindungen der Formel I in welchen $R_1$ Wasserstoff bedeutet, können in die entsprechenden (Halogen oder Acyl)-Derivate z. B. durch Halogenierung, vorzugsweise mit Chlor in Essigsäure oder unter Bedingungen der Friedel-Crafts Reaktion durch Acylierung mit einem Trihalogenacetyl-halogenid oder einer Halogensulfonsäure, und nachfolgende Behandlung mit einem Alkalimetall-niederalkoxid, -hydroxid oder -amid umgewandelt werden. Erhaltene Carbonsäure- oder Sulfonsäure-Derivate können dann in bekannter Weise, vorzugsweise unter alkalischen Bedingungen hydrolysiert und/oder mit Ammoniak Mono- oder Diniederalkylaminen amidiert werden. Die erhaltenen Carboxamide können nach konventionellen Methoden zu den entsprechenden Nitrilen hydratisiert werden.

Verbindungen der Formel I, in welchen $R_1$ Carboxy bedeutet, können beispielsweise erhalten werden, indem man Verbindungen, worin $R_1$ Cyan, Carbalkoxy oder Carbamoyl bedeutet, hydrolysiert.

Erhaltene tertiäre Amine, worin $R_2$ sich von Wasserstoff unterscheidet und beispielsweise $C_1$-$C_4$-Alkyl oder Phenyl-$C_1$-$C_3$-alkyl bedeutet, können in an sich bekannter Weise in die N-Oxide umgewandelt werden. Man arbeitet z. B. mit Wasserstoffsuperoxid oder organischen Persäuren, z. B. niederen Peralkansäuren oder Perbenzoesäuren, wie Peressigsäure oder m-Chlor-perbenzoesäure, vorzugsweise bei Zimmertemperatur oder mit der letztgenannten Säure, darunter, oder bis 100° mit verdünntem Wasserstoffperoxid, in Gegenwart von Niederalkansäuren, z. B. Essigsäure.

Schliesslich können die Verbindungen der Erfindung in Form von freien Basen oder als Salze erhalten werden. Eine erhaltene freie Base kann in das entsprechende Säureadditionssalz, vorzugsweise mit Säuren, kann man die therapeutisch verwendbare Säureadditionssalze ergeben oder wie die anderen Ionenaustauschern übergeführt werden. Erhaltene Salze können in die entsprechenden freien Basen, z. B. durch Behandlung mit einer stärkeren Base, wie mit einem Metallhydroxid oder Ammoniumhydroxid, basischen Salz oder einem Kationenaustauscher, z. B. mit einem Alkalimetallhydroxid oder -carbonat, umgewandelt werden. Säuren, die therapeutisch verwendbare Säureadditionssalze ergeben, sind z. B. die vorher beschriebenen anorganischen Säuren oder organischen Säuren.

Verbindungen der Formel I, in welchen $R_1$ Carboxy bedeutet, können auch in die entsprechenden Metall- oder Ammoniumsalze, durch Behandlung mit Alkali- oder Erdalkalimetallhydroxiden oder -carbonaten, Ammoniak oder mit vorhergenannten Aminen übergeführt werden.

Diese oder andere Salze, z. B. die Pikrate, können auch in der Reinigung von den erhaltenen freien Basen verwendet werden. Die Basen werden in ihre Salze übergeführt, die Salze abgetrennt und die Basen aus den Salzen freigesetzt.

Infolge der engen Beziehung zwischen den neuen Verbindungen in freier Form und in Form ihrer Salze sind im vorausgegangenen und nachfolgend unter freien Verbindungen und Salzen sinn- und zweckmässig gegebenenfalls auch die entsprechenden Salze bzw. freien Verbindungen zu verstehen.

Erhaltene Isomerengemische von Verbindungen, z. B. solchen der Formel I bis VII, können nach an sich bekannten Methoden, z. B. durch fraktionierte Destillation, Kristallisation und/oder Chromatographie, in die einzelnen Isomeren getrennt werden. Racemische Produkte können in die optischen Antipoden, z. B. bei Trennung ihrer diastereoisomeren Salze, z. B. durch fraktionierte Kristallisation der d- oder l-Tartrate getrennt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder anderen oben genannten Mitteln und/oder in einer inerten Atmosphäre, unter Kühlung bei Zimmertemperatur oder bei erhöhten Temperaturen, vorzugsweise beim Siedepunkt des verwendeten Lösungsmittels, bei normalem oder erhöhtem Druck durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird.

Im Verfahren der vorliegenden Erfindung werden vorteilhafterweise solche Ausgangsstoffe verwendet, welche zu den im vorstehenden als besonders wertvoll beschriebenen Verbindungen, insbesondere solchen der Formel II, führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen.

Vorzugsweise verwendet man Tabletten oder Gelatinekapseln, welche den Wirkstoff zusammen mit Verdünnungsmitteln, z. B. Laktose, Dextrose, Rohrzucker, Mannitol, Sorbitol, Cellulose und/oder Glycin, und Schmiermitteln, z. B. Kieselerde, Talk, Stearinsäure oder Salze davon, wie Magnesium- oder Calciumstearat, und/oder Polyäthlenglykol, aufweisen; Tabletten enthalten ebenfalls Bindemittel, z. B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, und/oder Brausemischungen, und Adsorptionsmittel, Farbstoffe, Geschmackstoffe und Süssmittel. Injizierbare Präparate sind vorzugsweise isotonische wässerige Lösungen oder Suspensionen und Suppositorien in erster Linie Fettemulsionen oder -suspensionen. Die pharmakologischen Präparate können sterilisiert sein und/oder Hilfsstoffe z. B. Konservier-, Stabilisier-, Netz- und/oder Emulgiermittel, Löslichkeitsvermittler, Salze zur Regulierung des osmotischen Druckes und/oder Puffer erhalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder Dragierverfahren, hergestellt und enthalten von etwa 0,1 % bis etwa 75 %, insbesondere von etwa 1 % bis etwa 50 % des Aktivstoffes. Einzeldosen für Säuger mit einem Gewicht von ungefähr 50 - 70 kg können zwischen ungefähr 18 - 100 mg des aktiven Bestandteils enthalten.

Die nachfolgenden Beispiele erläutern die Herstellung der neuen Verbindungen der Formel I, sollen jedoch den Umfang der Erfindung in keiner Weise beschränken. Die Temperaturen sind in Celsiusgraden angegeben.

**Beispiel 1:**

Eine Mischung von 6,94 g 1-{o-[5-Methyl-3-(1,2,4-triazolyl)methyl]-phenylcarbamoyl}-4-methylpiperazin, 52 ml Phosphoroxychlorid und 4,65 g Phosphorpentachlorid wird bei Raumtemperatur während 5 Stunden gerührt und dann zur Trockne eingedampft. Der Rückstand wird in 125 ml Methylenchlorid suspendiert, die Mischung wird auf 0° abgekühlt und dann werden 15,5 ml Triäthylamin tropfenweise zugesetzt. Die Mischung wird dann bei Raumtemperatur während 30 Minuten gerührt und mit kaltem Wasser gewaschen. Die wässrige Schicht wird mit Methylenchlorid gewaschen und die zusammengefügten organischen Schichten werden mit Wasser gewaschen, über Magnesiumsulfat getrocknet, mit Aktivkohle entfärbt und eingedampft. Der Rückstand wird mit 50 g Silicagel chromatographiert, wobei Methylenchlorid-Methanol-Ammoniumhydroxid (450 : 50 : 1) als Eluationsmittel verwendet wird, wobei als weniger polare Komponente 2-Methyl-5-(4-methyl-1-piperazinyl)-11H-1,2,4-triazol[2,3-c][1,3]-benzodiazepine erhalten wird.

Eine Lösung von 2-Methyl-5-(4-methyl-1-piperazinyl)-11H-1,2,4-triazolo[2,3-c][1,3]benzodiazepine in Isopropanol wird mit einer Lösung einer äquimolaren Menge Maleinsäure in Isopropanol behandelt, wobei 2-Methyl-5-(4-methyl-1-piperazinyl)-11H-1,2,4-triazolo[2,3-c][1,3]-benzodiazepin in Isopropanol behandelt, wobei 2-Methyl-5-(4-methyl-1-piperazinyl)-11H-1,2,4-traizolo[2,3c][1,3]benzodiazepinmonomaleat, mit einem Schmelzpunkt von 206 - 208°C erhalten wird. Diese Verbindung ist ein Salz einer Verbindung der Formel I, worin $R_1$ und $R_2$ = $CH_3$, $R_3$-$R_6$ = H und $C_nH_{2n}$ = $CH_2CH_2$ darstellt.

Das Ausgangsmaterial wird wie folgt hergestellt:

2250 ml absoluter Äthanol und 1500 g o-Nitrophenylacetonitrile werden in einer 22 Literflasche vorgegeben. Diese Suspension wird auf 5 - 10° abgekühlt und während 2,5 Stunden wird Chlorwasserstoffgas in die Mischung eingeleitet. Das Reaktionsgemisch wird bei 10° unter Stickstoffatmosphäre über Nacht gerührt. Danach wird es mit 16 000 ml Diäthyläther verdünnt und während 1 Stunde gerührt; der Feststoff wird durch Filtration abgetrennt, viermal mit 1000 ml Diäthyläther gewaschen und getrocknet (5 ml Hg/40°), wobei 2-(o-Nitrophenyl)-acetimidat-hydrochlorid vom Schmelzpunkt 122 - 123° (Zers.) erhalten wird.

Zu einer Lösung von 4,1 g Äthyl2-(o-nitrophenyl)-acetimidate hydrochloride in 40 ml Äthanol wird bei Raumtemperatur und über einen Zeitraum von 10 Minuten eine Lösung von Natriumäthoxid zugegeben, die durch Auflösen von 0,38 Natrium in 40 ml Äthanol entstanden ist. Die Mischung wird während 10 Minuten gerührt und filtriert. Dem Filtrat wird 1,29 g Acetylhydrazid zugegeben, die Mischung bei Raumtemperatur während 2 Stunden gerührt, die Feststoffe abfiltriert und mit Äthanol gewaschen, wobei die N-Acetyl-o-nitrophenylacetamidrazon vom Schmelzpunkt 195 - 197° erhalten wird.

Eine Mischung von 1,29 g N-Acetyl-o-nitrophenylacetamidrazon und 20 ml Äthanol wird während 60 Stunden am Rückfluß gekocht und dann zur Trockene eingedampft. Der ölige Rückstand wird aus Äther kristallisiert, wobei das 5-Methyl-3-o-nitrobenzyl)-1,2,4-triazol vom Schmelzpunkt 119 - 123°C erhalten wird.

Eine Mischung von 1.67 g 5-Methyl-3-(o-nitrobenzyl)-1,2,4-triazol, 42 mg 10 %-igem Palladium auf Aktivkohle und 15 ml Äthanol wird während 4 Stunden und einem Druck von 3 Atmosphären hydriert, filtriert, entfärbt, auf ein kleines Volumen eingedampft und mit Äther verdünnt, wobei 3-(o-Aminobenzyl)-5-methyl-1,2,4-triazol vom Schmelzpunkt 143 - 145°C erhalten wird.

Eine Mischung von 12,87 g 3-(o-Aminobenzyl)-5-methyl-1,2,4-triazol, 11.21 g 1,1'-Carbonyldiimidazol und 470 ml Methylenchlorid werden während 5 Tagen bei Raumtemperatur gerührt und dann filtriert. Die Filtrate werden mit Wasser gewaschen, über Magnesiumsulfat getrocknet, entfärbt und eingedampft. Der Rückstand wird aus Tetrahydrofuran umkristallisiert, wobei 2-Methyl-11H-1,2,4-triazol-[2,3-c][1,3]benzodiazepin-5-(6H)-on vom Schmelzpunkt 245 - 247° erhalten wird, d.h. das Ausgangsmaterial der Formel III, worin X = OH, $R_3$-$R_6$ = H, $R_1$ = $CH_3$ und $C_nH_{2n}$ = $CH_2CH_2$.

Eine Mischung von 7,23 g 2-Methyl-11H-1,2,4-triazo1[2,3-c][1,3]-benzodiazepin-5(6H)-on, 5,45 g N-Methylpiperazin und 76 ml Methylenchlorid wird während 48 Stunden am Rückfluß gekocht und zur Trockene eingedampft. Der Rückstand wird mit Silicagel chromatographiert unter Verwendung von Methylenchlorid-Methanol-Ammoniumhydroxid (150 : 50 : 1) als Eluationsmittel. Es wird 1-[o-[5-Methyl-3-(1,2,4-triazolyl)methyl]-phenylcarbamoyl]-4-methylpiperazin als schaumiges Material erhalten; Massenspektrum M+/e = 314; diese Verbindung stellt das Zwischenprodukt der Formel VI dar, worin $C_nH_{2n}$ = $CH_2CH_2$, $R_1$ und $R_2$ = $CH_3$, $R_3$-$R_6$ = H und Z = 0

**Beispiel 2:**

Ähnlich wie nach dem Verfahren von Beispiel 1 werden die folgenden Verbindungen der Formel I hergestellt, worin $C_nH_{2n}$ Äthylen, $R_2$ Methyl und $R_4$-$R_6$ Wasserstoff bedeutet.

| Verbindung | $R_1$ | $R_3$ | Salt | m.p. |
|---|---|---|---|---|
| 1 | $CH_2CH_3$ | H | Maleat | 196 - 199°C |
| 2 | H | H | Maleat | 202 - 204°C |
| 3 | H | 8-Cl | Maleat | 215 - 217°C |
| 4 | Cl | H | Maleat | 216 - 218°C |
| 5 | $SCH_3$ | H | Maleat | 209 - 212°C |
| 6 | $COOCH_2CH_3$ | H | Maleat | |

Die Ausgangsstoffe der Formel VI, worin $C_nH_{2n}$ Äthylen, $R_2$ Methyl, $R_4$-$R_6$-Wasserstoff und Z Oxo bedeuten, sind für die Verbindungen 1 bis 6 von Formel I die folgenden:

| Ausgangs-Material | $R_1$ | $R_3$ | Schmelzpunkt | Bemerkungen |
|---|---|---|---|---|
| 1/a | $CH_2CH_3$ | H | 153 - 159°C | |
| 2/a | H | H | 205 - 208°C | |
| 3/a | H | Cl | 182 - 186°C | |
| 4/a | Cl | H | 190 - 195°C | Cl para zur Triazolylmethylgruppe |
| 5/a | $SCH_3$ | H | 177 - 180°C | |
| 6/a | $COOCH_2CH_3$ | H | | |

Die Ausgangsmaterialien der Formel III, worin X OH und $R_4$-$R_6$ Wasserstoff bedeuten, für die Verbindungen 1 bis 6 der Formel I sind die folgenden:

| Ausgangs-material | $R_1$ | $R_3$ | Schmelzpunkt |
|---|---|---|---|
| 1/b | $CH_2CH_3$ | H | 165 - 178°C |
| 2/b | H | H | 133 - 136°C |
| 3/b | H | 8-Cl | 252 - 254°C |
| 4/b | Cl | H | |
| 5/b | $SCH_3$ | H | 250 - 252°C |
| 6/b | $COOCH_2CH_3$ | H | 216 - 220°C |

**Ausgangamaterialien für Verbindung 1:**

a) N-Propionyl-o-nitrophenylacetamidrazon, Schmelzpunkt 184.5 - 186.5°C.
b) 5-Äthyl-3-(o-nitrobenzyl)-1,2,4-triazol, Schmelzpunkt 100 - 103°C.
c) 5-Äthyl-3-(o-aminobenzyl)-1,2,4-triazol, Schmelzpunkt 148.5 - 151.5°C.

**Ausgangsmaterialien für Verbindung 2:**

a) N-Formyl-o-nitrophenylacetamidrazon, Schmelzpunkt 149 - 151°C, ausgehend von Formylhydrazid.
b) 3-(o-Nitrobenzyl)-1,2,4-triazol, Schmelzpunkt 123 - 125.

c) Zu einer Lösung von 9.64 g 3-(o-Nitrobenzyl)-1,2,4-triazol in 150 ml Tetrahydrofuran werden 220 ml einer 1,3M wässrigen Lösung von Titaniumtrichlorid zugefügt und die Mischung wird bei Raumtemperatur während 24 Stunden gerührt. Die Mischung wird in einem Eiswasserbad abgekühlt und es wird konzentriertes Ammoniumhydroxid tropfenweise zugefügt, bis das pH der Lösung 8 beträgt. Die Lösung wird dann weiter mit Wasser verdünnt und viermal mit Methylenchlorid extrahiert. Die kombinierten Extrakte werden mit Aktivkohle entfärbt, über Magnesiumsulfat getrocknet, auf ein kleines Volumen eingedampft und mit Äther verdünnt, wobei 3-(o-Aminobenzyl)-1,2,4-triazol vom Schmelzpunkt 134 - 136° C erhalten wird.

**Ausgangsmaterialien für Verbindung 3:**

a) N-Formyl-p-chlor-o-nitrophenylacetamidrazon, Schmelzpunkt 173 - 175° C (Zers.).
b) 3-(p-Chlor-o-nitrobenzyl)-1,2,4-triazol, Schmelzpunkt 180 - 185° C.
c) 3-(p-Chlor-o-aminobenzyl)-1,2,4-triazol, Schmelzpunkt 149 - 152° C.

**Ausgangsmaterialien für Verbindung 4:**

a) Zu einer Lösung von 2,36 g Äthylo-nitrophenylacetimidat in 23 ml Äthanol wird tropfenweise bei 0° C eine Lösung von Natriumäthoxid zugesetzt, die hergestellt worden ist durch Auflösen von 0,22 g Natrium in 11 ml Äthanol. Die Mischung wird während 30 Minuten gerührt, filtriert und dem Filtrat wird 1 g Ähylhydrazinocarbonat zugesetzt. Die Mischung wird bei Raumtemperatur während 60 Stunden gerührt, auf ein kleines Volumen eingedampft und filtriert, wobei N-Äthoxycarbonyl-o-nitrophenyl-acetamidrazon, Schmelzpunkt 183 - 185° C erhalten wird.

b) Eine Mischung von 1 g N-Äthoxycarbonyl-o-nitrophenylacetamidrazon und 10 ml Amylalkohol wird über Nacht am Rückfluß gekocht, gekühlt und filtriert, wobei 5-Hydroxy-3-(o-nitrobenzyl)-1,2,4-triazol vom Schmelzpunkt 210 - 212,5° C erhalten wird.

c) Eine Mischung von 1 g 5-Hydroxy-3-(o-nitrobenzyl)-1,2,4-triazol, 10 ml Phosphoroxychlorid und 1.8 g Phosphorpentachlorid wird zuerst bei 70° während 11 Stunden und dann bei Raumtemperatur über das Wochenende gerührt und dann zur Trockene eingedampft. Der Rückstand wird im Wasser aufgelöst, die Lösung mit 10 %-igem wässrigem Kaliumcarbonat basisch gestellt und dann dreimal mit Äthylacetat extrahiert. Die zusammengeschütteten Extrakte werden über Magnesiumsulfat getrocknet, mit Aktivkohle entfärbt und zur Trockene eingedampft. Der Rückstand wird mit 50 g Silicagel chromatographiert und zwar unter Verwendung von Methylenchlorid-Acetat (1 : 1) als Eluationsmittel, wobei 5-Chlor-3-(o-nitrobenzyl)-1,2,4-triazol, Schmelzpunkt 160 - 163° C erhalten wird.

d) Zu einer Lösung von 6,27 g 5-Clor-3-(o-nitrobenzyl)-1,2,4-triazol in 146 ml Tetrahydrofuran werden bei 0° unter Rühren 146 ml 20 %-ige wässrige Lösung von Titantrichlorid zugefügt. Die Mischung wird bei Raumtemperatur über Nacht gerührt, mit konzentriertem Ammoniumhydroxid basisch gestellt und dreimal mit Methylenchlorid extrahiert. Die zusammengeschütteten Extrakte werden getrocknet, eingedampft und der Rückstand aus Diäthyläther kristallisiert, wobei 3-(o-Aminobenzyl)-5-chloro-1,2,4-triazol vom Schmelzpunkt 162 - 165° C erhalten wird.

e) Eine Mischung von 0,35 g 3-(o-Aminobenzyl)-5-chloro-1,2,4-triazol, 9 ml Methylenchlorid und 0,284 g 1,1'-Carbonyldiimidazol wird bei Raumtemperatur über Nacht gerührt. Die Mischung wird auf ein kleines Volumen eingedampft und mit Diäthyläther verdünnt, wobei 1-{o-[5-Chloro-3-(1,2,4-triazolly)methyl]-phenylcarbamoyl}-imidazol vom Schmelzpunkt 165 - 167° C erhalten wird.

f) Eine Mischung von 0,324 g 1-{o-[5-Chlor-3-(1,2,4-triazolyl)-methyl]-phenylcarbamoyl}-imidazol, 3 ml Methylenchlorid und 0,11 g N-Methylpiperazin wird über Nacht bei Raumtemperatur gerührt. Die Mischung wird mit Wasser gewaschen, über Magnesiumsulfat getrocknet und eingedampft, wobei das 1-[o-[5-Chlor-3-(1,2,4-triazolyl)-methyl]-phenylcarbamoyl]-4-methylpiperazin vom Schmelzpunkt 190 - 195° C erhalten wird (Ausgangsmaterial 4/a).

**Ausgangsmaterialien für Verbindung 5:**

a) Zu einer Lösung von 4,1 g Äthyl-2-(o-nitrophenyl)acetimidat in 40 ml Äthanol wird eine Lösung von Natriumäthoxyd (hergestellt durch Auflösen von 0,38 g Natrium in 40 ml Äthanol) tropfenweise bei 0° C zugefügt. Nach Rühren bei Raumtemperatur während 30 Minuten werden die Feststoffe abfiltriert, das Filtrat wird auf ein Volumen von ungefähr 10 ml eingedampft und mit 20 ml Dimethylsulfoxid verdünnt. Der resultierenden Lösung werden 1,52 g Thiosemicarbazid zugefügt und die Mischung während 2 Tagen bei Raumtemperatur gerührt. Das Lösungsmittel wird im Vakuum abgedampft, der Rückstand mit Wasser und Äthylacetat behandelt; die wässrige Schicht wird noch einmal mit Äthylacetat extrahiert und die zusammengeschütteten organischen Extrakte werden mit Wasser gewaschen, über Magnesiumsulfat getrocknet, auf ein

kleines Volumen eingedampft und mit Diäthyläther verdünnt, wobei N-(Aminothiocarbonyl)-o-nitrophenylacetamidrazon vom Schmelzpunkt 172 - 174° C erhalten wird.

b) Eine Mischung von 1 g N-(Aminothiocarbonyl)-o-nitrophenylacetamidrazon und 10 ml Amylalkohol wird während 6 Stunden am Rückfluss gekocht, auf ein kleines Volumen eingedampft und mit Diäthyläther verdünnt, wobei 5-Mercapto-3-(o-nitrobenzyl)-1,2,4-triazol vom Schmelzpunkt 239 - 241° C erhalten wird.

c) Zu einer Suspension von 0,61 g Natriumhydrid in 50 ml Tetrahydrofuran werden 5 g Mercapto-3-(o-nitrobenzyl)-1,2,4-triazol portionenweise unter Rühren bei Raumtemperatur über einen Zeitraum von 30 Minuten zugegeben. Die Mischung wird während 2 Stunden gerührt, 1,51 ml Methyljodid werden auf einmal zugegeben; die Mischung wird über Nacht bei Raumtemperatur gerührt und dann im Vakuum zur Trockene eingedampft. Der Rückstand wird mit Wasser und Methylenchlorid behandelt; die wässrige Schicht wird noch einmal mit Methylenchlorid extrahiert; die zusammengeschütteten organischen Extrakte werden über Magnesiumsulfat getrocknet, auf ein kleines Volumen eingedampft und mit Diäthyläther verdünnt, wobei 5-Methylmercapto-3-(o-nitrobenzyl)-1,2,4-triazolo vom Schmelzpunkt 120 - 132° C erhalten wird.

d) Zu einer Lösung von 2 g 5-Methylmercapto-3-(o-nitrobenzyl)-1,2,4-triazol in 45 ml Tetrahydrofuran werden 44 ml 20 %-iges wässriges Titantrichlorid tropfenweise bei 0° zugefügt. Die Mischung wird bei Raumtemperatur über Nacht gerührt, mit Wasser verdünnt, auf 0° abgekühlt, mit Ammoniumhydroxid basisch gestellt und dreimal mit Methylenchlorid extrahiert. Die organischen Extrakte werden getrocknet, eingedampft und der Rückstand aus Toluol kristallisiert, wobei 3-(o-Aminobenzyl)-5-methylmercapto-1,2,4-triazol vom Schmelzpunkt 99 - 102° C erhalten wird.

e) Eine Mischung von 7 g 3-(o-Aminoenzyl)-5-methylmercapto-1,2,4-triazol 5,26 g 1,1'-Carbonyldiimidazol und 179 ml Methylenchlorid wird über Nacht unter Rückfluß erhitzt. Danach wird der Grossteil des Lösungsmittels im Vakuum abgedampft, die erhaltenen Feststoffe abfiltriert und mit einer kleinen Menge Methylenchlorid gewaschen, wobei 2-Methylmercapto-11H-1,2,4-triazo1[2,3-c][1,3]benzodiazepine-5(6H)-on vom Schmelzpunkt 250 - 252° C erhalten wird (Ausgangsmaterial 5/b).

**Ausgangsmaterialien für Verbindung 6:**

a) Zu einer Lösung von 1,86 g Äthyl-2-(o-nitrophenyl)acetimidate in 19 ml Äthanol wird eine Lösung von Natriumäthoxid (hergestellt durch Auflösen von 0,17 g Natrium in 8,7 ml Äthanol) tropfenweise bei 0° zugegeben. Nach Rühren der Mischung während 30 Minuten werden die Feststoffe abfiltriert, 1 g Äthyloxalylhydrazid wird zugegeben und die Mischung wird während 48 Stunden bei Raumtemperatur gerührt. Das Produkt wird abfiltriert und mit Äthanol gewaschen, wobei N-Carboäthoxycarbonyl-o-nitrophenylacetamidrazon vom Schmelzpunkt 136 - 138° C erhalten wird.

b) Eine Mischung von 23.97 g N-Carboäthoxycarbonyl-o-nitrophenylacetamidrazone und 240 ml Äthanol wird über Nacht am Rückfluß gekocht, filtriert und eingedampft. Der Rückstand wird mit Silicagel chromatographiert, wobei Methylenchlorid-Methanol-Ammoniumhydroxid (300 : 50 : 1) als Eluationsmittel verwendet wird. Es wird 5-Äthoxycarbonyl-3-(o-nitrobenzyl)-1,2,4-triazol vom Schmelzpunkt 135 - 140° C erhalten.

c) Eine Mischung von 1 g 5-Äthoxycarbonyl-3-(o-nitrobenzyl)-1,2,4-triazol, 10 ml Äthanol und 25 mg Platinoxid wird während 3 Stunden unter einem Druck von 3 Atmosphären hydriert, filtriert und eingedampft, wobei 3-(o-Aminobenzyl)-5-äthoxycarbonyl-1,2,4-triazol erhalten wird.

d) Eine Mischung von 13,96 g 3-(o-Aminobenzyl)-5-äthoxycarbonyl-1,2,4-triazol, 410 ml Methylenchlorid und 9,19 g 1,1'-Carbonyldiimidazol wird bei Raumtemperatur während 2 Stunden gerührt und dann filtriert. Das Filtrat wird mit Wasser gewaschen, über Magnesiumsulfat getrocknet, mit Aktivkohle entfärbt, auf ein kleines Volumen eingedampft und mit Diäthyläther verdünnt. Das erhaltene Produkt wird abfiltriert und aus Tetrahdrofuran kristallisiert, wobei 2-Äthoxycarbonyl-11H-1,2,4-triazol[2,3-c][1,3]benzodiazepin-5-(6H)-on vom Schmelzpunkt 216 - 220° C erhalten wird (Ausgangsmaterial 6/b).

**Beispiel 3:**

Zu einer Lösung von 6,25 g 2-Methyl-5-cyanthio-11H-1,2,4-triazol-[2,3-c][1,3]benzodiazepin in 7,3 ml Hexamethylphosphoramid werden bei 0° tropfenweise 4,94 g N-Methylpiperazin über einen Zeitraum von 5 Minuten zugegeben. Die Mischung wird bei Raumtemperatur während 4 Stunden gerührt, in Wasser gegossen und mit Äthylacetat extrahiert. Die organischen Extrakte werden mit Wasser gewaschen, über Magnesiumsulfat getrocknet und zur Trockene eingedampft, wobei 2-Methyl-5-(4-methyl-1-piperazinyl)-11H)-1,2,4-triazol[2,3-c][1,3]benzodiazepin vom Schmelzpunkt 185 - 187° C erhalten wird. Dieses Material wird mit einer äquivalenten Menge Maleinsäure behandelt, wobei 2-Methyl-5-(4-methyl-1-piperazinyl)-11H-1,2,4-triazol[2,3-c][1,3]benzodiazepinmonomaleat gemäß Beispiel 1 erhalten wird.

Das Ausgangsmaterial wird wie folgt hergestellt:

Zu einer Lösung von 5,41 g Thiophosgen in 30 ml Methylenchlorid wird bei 0° tropfenweise eine Lösung von 7,5 g 5-Methyl-3-(o-aminobenzyl)-1,2,4-triazol und 8,07 g Triäthylamin in 285 ml Methylenchlorid während eines Zeitraumes von 45 Minuten zugegeben. Die Mischung wird bei Raumtemperatur über Nacht gerührt, zuerst mit

10 %-igem wäßrigem Kaliumbicarbonat und dann mit Wasser gewaschen, über Magnesiumsulfat getrocknet, mit Aktivkohle entfärbt und zur Trockene eingedampft, wobei 5-Methyl-3-(o-isothiocyanatobenzyl)-1,2,4-triazol erhalten wird; IR 2080 $cm^{-1}$.

Zu einer Suspension von 0,91 g Natriumhydrid in 27 ml Tetrahydrofuran wird tropfenweise eine Lösung von 8,74 g 5-Methyl-3-(o-isothiocyanatobenzyl)-1,2,4-triazol in 60 ml Tetrahydrofuran während eines Zeitraumes von 20 Minuten hinzugefügt. Die Mischung wird bei Raumtemperatur während 2 Stunden gerührt. Zu der entstandenen Suspension wird eine Lösung von 4,02 g Cyanogenbromid in 35 ml Tetrahydrofuran tropfenweise bei 0° über einen Zeitraum von 15 Minuten hinzugefügt. Die Mischung wird bei 0° während 1,5 Stunden gerührt, in Wasser gegossen und dann dreimal mit Äthylacetat extrahiert. Die organischen Extrakte werden über Magnesiumsulfat getrocknet, mit Aktivkohle entfärbt und eingedampft, wobei 2-Methyl-5-cyanothio-11H-1,2,4-triazol[2,3-c][1,3]benzodiazepin erhalten wird; IR 2150 $cm^{-1}$.

**Beispiel 4:**

Zu einer Lösung von 1,0 g 2-Methyl-5-(4-äthoxycarbonyl-1-piperazinyl)-11H-1,2,4-triazol[2,3-c][1,3]benzodiazepin in 10 ml trockenem Tetrahydrofuran werden 500 mg Lithiumaluminiumhydrid auf einmal zugefügt und die Mischung dann unter Stickstoffatmosphäre während 24 Stunden am Rückfluß gekocht. Die Mischung wird auf 0° abgekühlt, der Überschuß an Lithiumaluminiumhydrid wird mit Äthylacetat zerstört, die Mischung dann in Wasser gegossen und mit Äthylacetat extrahiert. Die Extrakte werden getrocknet und eingedampft, wobei nach Reinigung 2-Methyl-5-(4-methyl-1-piperazinyl)-11H-1,2,4-triazo1[2,3-c][1,3]benzodiazepin gemäß Beispiel 1 erhalten wird.

Das Ausgangsmaterial wird gemäß den Verfahrensangaben von Beispiel 3 durch Kondensation von 2-Methyl-5-cyanothio-11H-1,2,4-triazol[2,3-c][1,3]benzodiazepin mit N-Carboäthoxypiperazin erhalten.

**Beispiel 5:**

Zu einer Lösung von 200 mg 2-Methyl-5-(4-benzyloxycarbonyl-1-piperazinyl)-11H-1,2,4-triazol[2,3-c][1,3]benzodiazepin in 0,6 ml Essigsäure werden 0,70 ml einer 2N Lösung von Bromwasserstoffsäure in Essigsäure zugefügt. Die Mischung wird während 2 Stunden auf 100°C erhitzt und dann bei Raumtemperatur über Nacht gerührt. Das Aufarbeiten ergibt 2-Methyl-5-(4H-1-piperazinyl)-1H-1,2,4-triazo1[2,3-c][1,3]benzodiazepin.

Das Ausgangsmaterial wird ähnlich wie das Ausgangsmaterial von Beispiel 4 hergestellt durch Ersatz von 1-Äthoxycarbonylpiperazin mit der äquivalenten Menge von 1-Benzyloxycarbonylpiperazin.

**Beispiel 6:**

Eine Mischung von 300 mg 5-(4H-1-Piperazinyl)-11H-1,2,4-triazo1[2,3-c][1,3]benzodiazepin, 0,5 g Kaliumcarbonat, 1 Moläquivalent Methyljodid und 2 ml Aceton wird über Nacht bei Raumtemperatur gerührt und dann eingedampft. Dem Rückstand wird Wasser zugefügt und die Mischung wird mit Methylenchlorid extrahiert. Die Extrakte werden über Magnesiumsulfat getrocknet, eingedampft und der Rückstand wird gereinigt, wobei 2-Methyl-5-(4-methyl-1-piperazinyl)-11H-1,2,4-triazol[2,3-c][1,3]benzodiazepin gemäß Beispiel 1 erhalten wird.

**Beispiel 7:**

Zu einer Lösung von 5 g 2-Methyl-5-(4-methyl-1-piperazinyl)-11H-1,2,4-triazo1[2,3-c][1,3]benzodiazepin in 50 ml Methylenchlorid werden portionenweise 3,75 g m-Chlorperbenzoesäure bei 0° unter Rühren zugegeben. Die Mischung wird dann bei Raumtemperatur über Nacht gerührt und zur Trockene eingedampft. Der schaumige Rückstand passiert dann 100 g Amberlite IRA-400 (Ionenaustauscherharz), wobei Wasser als Eluationsmittel dient. Das Verdampfen des Eluationsmittels ergibt 2-Methyl-5-(4-methyl-4-oxido-1-piperazinyl)-11H-1,2,4-triazol[2,3-c] [1,3]benzodiazepin.

**Beispiel 8:**

Die folgenden Verbindungen werden hergestellt gemäß den Methoden, wie sie in den vorausgehenden Beispielen illustriert sind und werden erhalten aus äquivalenten Mengen der entsprechend substituierten Ausgangsmaterialien.

a) 2-Methyl-5-(4-benzyl-1-piperazinyl)-11H-1,2,4-triazo1[2,3-c]-[1,3]benzodiazepin;
b) 2-Methyl-5-(4-allyl-1-piperazinyl)-11H-1,2,4-triazol[2,3-c-]-[1,3]benzodiazepin;
c) 2-Methly-5-(4-methyl-1-homopiperazinyl)-11H-1,2,4-triazol(2,3-c]-[1,3]benzodiazepin;
d) 2,8-Dimethyl-5-(4-methyl-1-piperazinyl)-11H-1,2,4-triazol[2,3-c]-[1,3]enzodiazepin;
e) 8-Fluor-2-methyl-5-(4-methyl-1-piperazinyl)-11H-1,2,4-triazol-[2,3-c][1,3]benzodiazepin;
f) 8-Methoxy-2-methyl-5-(4-methyl-1-piperazinyl)-11H-1,2,4-triazol-[2,3-c][1,3]benzodiazepin;
g) 2-Methyl-5-(4-hydroxyethyl-1-piperazinyl)-11H-1,2,4-triazol-[2,3-c][1,3]benzodiazepin.

**Beispiel 9:**

5100 ml Amylalcohol und 918,35 g N-Methylpiperazin werden in einem 12 Liter Dreihalskolben vorgelegt, der mit einem Dean-Stark Adapter ausgerüstet ist. Die Lösung wird unter Stickstoffatmosphäre gerührt, wobei 989 ml 10N äthanolische Chlorwasserstofflösung schnell zugegeben wird. Das Reaktionsgemisch wird am Rückfluß erhitzt und das Destillat wird im Dean-Stark Adapter aufgefangen. Wenn die Temperatur der Reaktionsmischung 131° erreicht, wird der Dean-Stark Adapter entfernt und eine zusätzliche Menge von 918,35 g N-Methylpiperidin gefolgt von 1112,6 g 2-Methyl-5-methylthio-11H-1,2,4-triazol[2,3-c][1,3]benzodiazepin wird zugegeben. Die Mischung wird unter Stickstoffatmosphäre während 20 Stunden unter Rückfluß erhitzt. Amylalkohol wird dann unter reduziertem Druck bei einer Wasserbadtemperatur von 80°C entfernt. Das viskose, verbleibende Öl wird in 10 000 ml Methylenchlorid aufgelöst, dreimal mit 4000 ml 4N Natriumhydroxyd und sechsmal mit 4000 ml Wasser gewaschen. Die Methylenchloridlösung wird dann dreimal mit 2000 ml 6N Chlorwasserstoffsäure gewaschen. Die wässrige Lösung wird zweimal mit 2000 ml Methylenchlorid gewaschen, mit Aktivkohle entfärbt und filtriert. Das wässrige Filtrat wird mit 1500 ml einer wässrigen Ammoniumhydroxidlösung auf ein pH 9 - 10 eingestellt. Das abgetrennte Öl wird dreimal mit 4000 ml Äthylenchlorid extrahiert, die Extrakte werden über 1000 g Natriumsulfat getrocknet und das Lösungsmittel wird entfernt, wobei 2-Methyl-5-(4-methyl-1-piperazinyl)-11H-1,2,4-triazol[2,3-c][1,3]benzodiazepin gemäss Beispiel 1 erhalten wird. Behandlung mit einer äquimolaren Menge von Maleinsäure in Isopropanol ergibt 2-Methyl-5-(4-methyl-1-piperazinyl)-11H-1,2,4-triazol[2,3-c][1,3]benzodiazepinmonomaleat gemäß Beispiel 1, Schmelzpunkt 206 - 208°C.

Das Ausgangsmaterial wird wie folgt hergestellt:

Zu einer Suspension von 0,91 g Natriumhydrid in 27 ml Tetrahydrofuran wird tropfenweise eine Lösung von 8,74 g 5-Methyl-3-(o-isothiocyanatobenzyl)-1,2,4-triazol in 60 ml Tetrahydrofuran über einen Zeitraum von 20 Minuten zugegeben. Die Mischung wird während 2 Stunden bei Raumtemperatur gerührt. Der resultierenden Suspension wird eine Lösung von 5,4 g Methyljodid in 35 ml Tetrahydrofuran tropfenweise bei 0° über einen Zeitraum von 15 Minuten hinzugefügt. Die Mischung wird bei Raumtemperatur während 1 Stunde gerührt, dann in Wasser gegossen und dreimal mit Methylenchlorid extrahiert. Die organischen Extrakte werden über Magnesiumsulfat getrocknet, mit Aktivkohle entfärbt und eingedampft, wobei 2-Methyl-5-methylthio-11H-1,2,4-triazol[2,3-c][1,3]benzodiazepin erhalten wird.

2-Methyl-5-methylthio-11H-1,2,4-triazol[2,3-c][1,3]benzodiazepin wird auch wie folgt erhalten:

Eine Suspension von 8 g 5-Methyl-3-(o-isothiocyantobenzyl)-1,2,4-triazol in 100 ml Toluol wird über Nacht am Rückfluß gekocht und auf Raumtemperatur abgekühlt, wobei 2-Methyl-11H-1,2,4-triazol-[2,3-c][1,3]benzodiazepin-5(6H)-thion erhalten wird. Eine Behandlung mit Methyljodid wie oben beschrieben ergibt 2-Methyl-5-methylthio-11H-1,2,4-triazol[2,3-c][1,3]benzodiazepin.

**Beispiel 10:**

Herstellung von 10 000 Tabletten mit einem Gehalt von je 25 mg der aktiven Substanz:

**Bestandteile:**

| | |
|---|---|
| 2-Methyl-5-(4-methyl-1-piperazinyl)-11H-1,2,4-triazol[2,3-c][1,3]benzodiazepinmonomaleat | 250.00 g |
| Milchzucker | 957.00 g |
| Maisstärke | 75.00 g |
| Polyäthylenglykol 6'000 | 75.00 g |
| Talkpulver | 75.00 g |
| Magnesiumstearat | 18.00 g |
| gereinigtes Wasser | q.s. |

**Verfahren:**

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granualt wird über Nacht bei 35°C getrocknet, durch ein Sieb von 1,2 ml Maschenweite getrieben und zu Tabletten mit 6,4 ml Durchmesser, welche eine Bruchrille aufweisen, gepresst.

**Beispiel 11:**

Herstellung von 10 000 Kapseln mit einem Gehalt von je 10 mg der aktiven Substanz:

**Bestandteile:**

| | |
|---|---|
| 2-Methyl-5-(4-methyl-1-piperazinyl)-11H-1,2,4-triazol[2,3-c][1,3]benzodiazepinmonomaleat | 100.0 g |
| Milchzucker | 1 800.0 g |
| Talkpulver | 100.0 g |

**Verfahren:**

Sämtliche pulverigen Bestandteile werden mit einem Sieb von 0,6 mm Maschenweite gesiebt. Dann wird der Wirkstoff zuerst mit Talk und darauffolgend mit Milchzucker in einem geeigneten Mischer homogenisiert. Kapseln Nr. 3 werden mit je 200 mg des Gemisches mit mit einer Füllmaschine gefüllt. In analoger Weise werden Tabletten oder Kapseln von anderen erfindungsgemäßen Verbindungen, z. B. solchen, die in weiteren Beispielen illustriert sind, hergestellt.

**Patentansprüche** für die Vertragsstaaten: BE, CH, DE, FR, GB, IT, LI, LU, NL, SE

1. Verbindungen der Formel

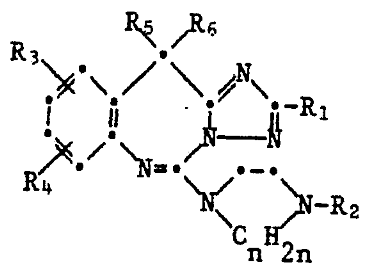

(I),

worin $R_1$ Wasserstoff, $C_1$-$C_4$-Alkylthio, Amino, ($C_1$-$C_4$-Alkanoyl-, $C_1$-$C_4$-Alkoxycarbonyl-, Carbamoyl-, Sulfamoyl-, $C_1$-$C_4$-Monoalkyl-, oder Di-$C_1$-$C_4$-alkylcarbamoyl- oder -sulfamoyl-, Halosulfonyl-, Phenyl-$C_1$-$C_2$-alkoxycarbonyl-, $C_1$-$C_4$-Alkyl- oder Di-$C_1$-$C_4$-alkyl)-amino, $C_1$-$C_4$-Alkoxy, ($C_1$-$C_4$-Alkanoyl-, $C_1$-$C_4$-Alkoxycarbonyl-, Carbamoyl-, Sulfamoyl-, mono-$C_1$-$C_4$-Alkyl- oder di-$C_1$-$C_4$-Alkylcarbamoyl- oder sulfamoyl-, Halosulfonyl- oder Phenyl-$C_1$-$C_2$-alkoxycarbonyl-)oxy, $C_1$-$C_4$-Alkyl, Acetyl, Propionyl, Hydroxy, Halogen, Trifluormethyl, Cyano, Carboxy, Methoxy- oder Äthoxycarbonyl, Carbamoyl, Mono- oder Dimethyl- oder -äthylcarbamoyl, Hydroxy-$C_1$-$C_4$-alkyl oder Di-$C_1$-$C_4$-alkylamino-$C_1$-$C_4$-alkyl bedeutet;

$C_nH_{2n}$ für $C_2$-$C_4$-Alkylen steht, welches die zwei Stickstoffatome durch 2 oder 3 Kohlenstoffatome trennt;

$R_2$ für Wasserstoff, $C_1$-$C_7$-Alkyl, Allyl, Propargyl, Acetyl, Propionyl, Phenyl-$C_1$-$C_3$-alkyl, welches am Phenylring durch Halogen, $C_1$-$C_4$-Alkoxy oder $C_1$-$C_4$-Alkyl substituiert sein kann, Methoxy- oder Äthoxycarbonyl, Phenylmethoxycarbonyl, Phenyläthoxycarbonyl, 2-Hydroxy-(äthyl oder propyl), 3-Hydroxy-(propyl oder butyl), 4-Hydroxybutyl, $C_2$-$C_4$-Alkanoyloxy-$C_2$-$C_4$-alkyl, Phenoxy-$C_2$-$C_4$-alkyl, welches am Phenylring durch Halogen, $C_1$-$C_4$-Alkoxy oder $C_1$-$C_4$-Alkyl substituiert sein kann, oder $C_1$-$C_4$-Alkoxy-$C_2$-$C_4$-alkyl;

$R_3$ und $R_4$ unabhängig voneinander Wasserstoff, $C_1$-$C_7$-Alkyl, $C_1$-$C_4$-Alkoxy, $C_1$-$C_4$-Alkylthio, Halogen,

Trifluormethyl, Hydroxy, Acetoxy, Propionyloxy, Sulfamoyl, Mono- oder Di-$C_1$-$C_4$-alkylsulfamoyl und $R_5$ und $R_6$ Wasserstoff oder $C_1$-$C_4$-Alkyl bedeuten; ihre N-Oxide, und Salze.

2. Verbindungen gemäss Anspruch 1 der Formel I, worin $R_1$ Wasserstoff, $C_1$-$C_4$-Alkyl, Halogen, Trifluormethyl, $C_1$-$C_4$-Alkyloxy oder $C_1$-$C_4$-Alkylthio bedeutet; $C_nH_{2n}$ für $C_2$-$C_4$-Alkylen steht, welches die zwei Stickstoffatome durch 2 oder 3 C-Atome trennt, $R_2$ für Wasserstoff, $C_1$-$C_4$-Alkyl, Methoxycarbonyl, Äthoxycarbonyl oder Hydroxy-$C_2$-$C_4$-alkyl, $R_3$ Wasserstoff, $C_1$-$C_4$-Alkyl, $C_1$-$C_4$-Alkoxy, $C_1$-$C_4$-Alkylthio, Halogen oder Trifluormethyl bedeutet, und $R_4$ für Wasserstoff steht und $R_5$ und $R_6$ die Bedeutung von Wasserstoff oder $C_1$-$C_4$-Alkyl haben; ihre N-Oxide und Salze.

3. Verbindungen gemäss Anspruch 1 der Formel I, worin $R_1$ Wasserstoff, Methyl, Äthyl, Methylthio, Chlor, Methoxy oder Trifluormethyl bedeutet; n für die ganze Zahl 2 oder 3 steht; $R_2$ für Wasserstoff, $C_1$-$C_3$-Alkyl, Methoxycarbonyl, Äthoxycarbonyl, Hydroxyäthyl oder Hydroxypropyl steht; $R_3$ Wasserstoff, Methyl, Methoxy, Methylthio, Chlor oder Trifluormethyl; $R_4$ Wasserstoff und $R_5$ und $R_6$ Wasserstoff oder Methyl bedeuten; ihre N-Oxide und Salze.

4. Verbindungen gemäss Anspruch 1 der Formel II

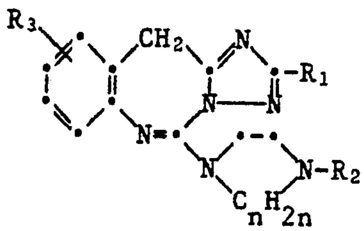

(II),

worin $R_1$ Wasserstoff, Halogen, Trifluormethyl, $C_1$-$C_4$-Alkylmercapto, $C_1$-$C_4$-Alkoxy, oder $C_1$-$C_4$-Alkyl bedeutet; $R_2$ für Wasserstoff, $C_1$-$C_4$-Alkyl oder Hydroxy-$C_2$-$C_4$-alkyl steht, worin Hydroxy vom Stickstoffatom durch mindestens 2 Kohlenstoffatome getrennt ist; $R_3$ Wasserstoff, $C_1$-$C_4$-Alkyl, $C_1$-$C_4$-Alkoxy, $C_1$-$C_4$-Alkylthio, Halogen oder Trifluormethyl bedeutet; $C_nH_{2n}$ für Äthylen oder Propylen steht; ihre N-Oxide und Salze.

5. Verbindungen gemäss Anspruch 1 der Formel II, worin $R_1$ Wasserstoff, Halogen, $C_1$-$C_4$-Alkylthio, $C_1$-$C_4$-Alkoxy oder $C_1$-$C_4$-Alkyl; $R_2$ Wasserstoff, $C_1$-$C_4$-Alkyl oder Hydroxy-$C_2$-$C_4$-alkyl bedeuten, worin Hydroxy vom Stickstoffatom durch 2 oder 3 Kohlenstoffatome getrennt ist; $R_3$ für Wasserstoff, $C_1$-$C_4$-Alkyl, Halogen oder Trifluormethyl steht; $C_nH_{2n}$ Äthylen bedeutet, ihre N-Oxide und therapeutisch verwendbaren Salze davon.

6. Verbindungen gemäss Anspruch 1 der Formel II, worin $R_1$ Wasserstoff, Methyl, Äthyl, Chlor, Methylthio oder Methoxy; $R_2$ Wasserstoff, Methyl, Äthyl, Propyl, 2-Hydroxyäthyl oder 3-Hydroxypropyl; $R_3$ Wasserstoff, Methyl, Methoxy, Fluor, Chlor oder Trifluormethyl bedeutet; und $C_nH_{2n}$ Äthylen bedeutet, und therapeutisch verwendbaren Salze.

7. 2-Methyl-5-(4-methyl-1-piperazinyl)-11H-1,2,4-triazol[2,3-c]-[1,3]benzodiazepin und seine therapeutisch verwendbaren Salze.

8. Pharmazeutische Präparate enthaltend Verbindungen der Formel I gemäss Anspruch 1, ihre N-Oxide und Salze, zusammen mit einem pharmazeutischen Trägermaterial.

9. Die im Anspruch 1 genannten Verbindungen zur Anwendung in einem Verfahren zur therapeutischen Behandlung des menschlichen oder tierischen Körpers.

10. Die im Anspruch 1 genannten Verbindungen als neuroleptische Mittel.

11. Die im Anspruch 1 genannten Verbindungen als antihistaminische Mittel.

12. Verwendung der im Anspruch 1 genannten Verbindungen zur Herstellung von pharmazeutischen Präparaten.

13. Verfahren zur Herstellung von Verbindungen der Formel I gemäss Anspruch 1, dadurch gekennzeichnet, dass man

a) eine Verbindung der Formel III

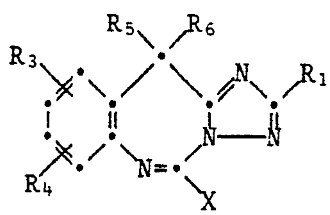

(III),

worin X eine zusammen mit Wasserstoff oder einem Alkalimetall abspaltbare Gruppe bedeutet und die anderen Symbole die unter der Formel I angegebenen Bedeutungen haben, mit einer Verbindung der Formel IV

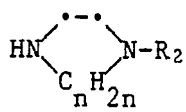

(IV)

oder einem Alkalimetallderivat davon, in welcher $R_2$ die unter der Formel I angegebene Bedeutung hat, kondensiert, oder

b) eine Verbindung der Formel VI

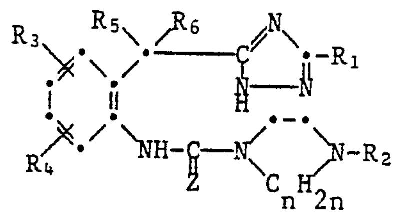

(VI),

worin Z ein Sauerstoff- oder Schwefelatom oder NH bedeutet und die anderen Symbole die oben angegebenen Bedeutungen haben, unter entwässernden, dehydrosulfurierenden oder desaminierenden Bedingungen ringschliesst und, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomere oder Razemate auftrennt, und/oder, wenn erwünscht, erhaltene Razemate in die optischen Antipoden aufspaltet.

**Patentansprüche** für den Vertragsstaat AT

1. Verfahren zur Herstellung von Verbindungen der Formel

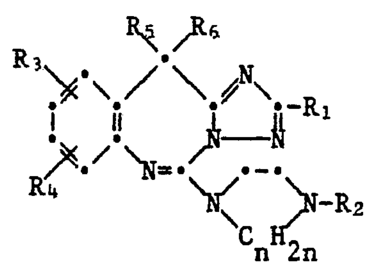

(I),

worin $R_1$ Wasserstoff, $C_1$-$C_4$-Alkylthio, Amino, ($C_1$-$C_4$-Alkanoyl-, $C_1$-$C_4$-Alkoxycarbonyl-, Carbamoyl-, Sulfamoyl-, $C_1$-$C_4$-Monoalkyl-, oder Di-$C_1$-$C_4$-alkylcarbamoyl- oder -sulfamoyl-, Halosulfonyl-, Phenyl-$C_1$-$C_2$-alkoxycarbonyl-, $C_1$-$C_4$-Alkyl- oder Di-$C_1$-$C_4$-alkyl)-amino, $C_1$-$C_4$-Alkoxy, ($C_1$-$C_4$-Alkanoyl-, $C_1$-$C_4$-Alkoxycarbonyl-, Carbamoyl-, Sulfamoyl-, mono-$C_1$-$C_4$-Alkyl- oder di-$C_1$-$C_4$-Alkylcarbamoyl- oder -sulfamoyl-, Halosulfonyl- oder Phenyl-$C_1$-$C_2$-alkoxycarbonyl-)oxy, Acyloxy, $C_1$-$C_4$-Alkyl, Acetyl, Propionyl, Hydroxy, Halogen, Trifluormethyl, Cyano, Carboxy, Methoxy- oder Äthoxycarbonyl, Carbamoyl, Mono- oder Dimethyl- oder -äthylcarbamoyl, Hydroxy-$C_1$-$C_4$-alkyl oder Di-$C_1$-$C_4$-alkylamino-$C_1$-$C_4$-alkyl bedeutet;

$C_nH_{2n}$ für $C_2$-$C_4$-Alkylen steht, welches die zwei Stickstoffatome durch 2 oder 3 Kohlenstoffatome trennt;

$R_2$ für Wasserstoff, $C_1$-$C_7$-Alkyl, Allyl, Propargyl, Acetyl, Propionyl, Phenyl-$C_1$-$C_3$-alkyl, welches am Phenylring durch Halogen, $C_1$-$C_4$-Alkoxy oder $C_1$-$C_4$-Alkyl substituiert sein kann, Methoxy- oder Äthoxycarbonyl, Phenylmethoxycarbonyl, Phenyläthoxycarbonyl, 2-Hydroxy-(äthyl oder propyl), 3-Hydroxy-(propyl oder butyl), 4-Hydroxybutyl, $C_2$-$C_4$-Alkanoyloxy-$C_2$-$C_4$-alkyl, Phenoxy-$C_2$-$C_4$-alkyl, welches am Phenylring durch Halogen, $C_1$-$C_4$-Alkoxy oder $C_1$-$C_4$-Alkyl substituiert sein kann, oder $C_1$-$C_4$-Alkoxy-$C_2$-$C_4$-alkyl;

$R_3$ und $R_4$ unabhängig voneinander Wasserstoff, $C_1$-$C_7$-Alkyl, $C_1$-$C_4$-Alkoxy, $C_1$-$C_4$-Alkylthio, Halogen, Trifluormethyl, Hydroxy, Acetoxy, Propionyloxy, Sulfamoyl, Mono- oder Di-$C_1$-$C_4$-alkylsulfamoyl und $R_5$ und $R_6$ Wasserstoff oder $C_1$-$C_4$-Alkyl bedeuten; ihren N-Oxiden, und Salzen, dadurch gekennzeichnet, daß man

a) eine Verbindung der Formel III

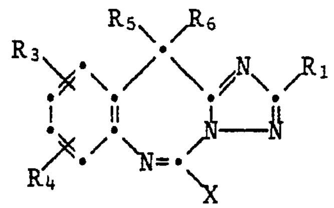

(III),

worin X eine zusammen mit Wasserstoff oder einem Alkalimetall abspaltbare Gruppe bedeutet und die anderen Symbole die unter der Formel I angegebenen Bedeutungen haben, mit einer Verbindung der Formel IV

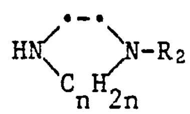

(IV)

oder einem Alkalimetallderivat davon, in welcher $R_2$ die unter der Formel I angegebene Bedeutung hat, kondensiert, oder

b) eine Verbindung der Formel VI

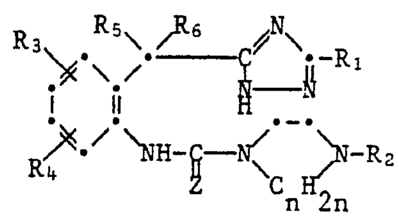

(VI),

worin Z ein Sauerstoff- oder Schwefelatom oder NH bedeutet und die anderen Symbole die oben angegebenen Bedeutungen haben, unter entwässernden, dehydrosulfurierenden oder desaminierenden Bedingungen ringschliesst und, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomere oder Razemate auftrennt, und/oder, wenn erwünscht, erhaltene Razemate in die optischen Antipoden aufspaltet.

2. Verfahren gemäss Anspruch 1, dadurch gekennzeichnet, dass man Verbindungen der Formel I, worin $R_1$ Wasserstoff, $C_1$-$C_4$-Alkyl, Halogen, Trifluormethyl, $C_1$-$C_4$-Alkyloxy oder $C_1$-$C_4$-Alkylthio bedeutet; $C_n H_{2n}$ für $C_2$-$C_4$-Alkylen steht, welches die zwei Stickstoffatome durch 2 oder 3 C-Atome trennt, $R_2$ für Wasserstoff, $C_1$-$C_4$-Alkyl, Methoxycarbonyl, Äthoxycarbonyl oder Hydroxy-$C_2$-$C_4$-alkyl, $R_3$ Wasserstoff, $C_1$-$C_4$-Alkyl, $C_1$-$C_4$-Alkoxy, $C_1$-$C_4$-Alkylthio, Halogen oder Trifluormethyl bedeutet, und $R_4$ für Wasserstoff steht und $R_5$ und $R_6$ die Bedeutung von Wasserstoff oder $C_1$-$C_4$-Alkyl haben; ihre N-Oxide und Salze herstellt.

3. Verfahren gemäss Anspruch 1, dadurch gekennzeichnet, dass man Verbindungen der Formel I, worin $R_1$ Wasserstoff, Methyl, Äthyl, Methylthio, Chlor, Methoxy oder Trifluormethyl bedeutet; n für die ganze Zahl 2 oder 3 steht; $R_2$ für Wasserstoff, $C_1$-$C_3$-Alkyl, Methoxycarbonyl, Äthoxycarbonyl, Hydroxyäthyl oder Hydroxypropyl steht; $R_3$ Wasserstoff, Methyl, Methoxy, Methylthio, Chlor oder Trifluormethyl; $R_4$ Wasserstoff und $R_5$ und $R_6$ Wasserstoff oder Methyl bedeuten; ihre N-Oxide und Salze herstellt.

4. Verfahren gemäss Anspruch 1, dadurch gekennzeichnet, dass man Verbindungen der Formel II

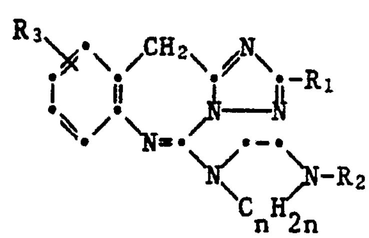

(II),

worin $R_1$ Wasserstoff, Halogen, Trifluormethyl, $C_1$-$C_4$-Alkylmercapto, $C_1$-$C_4$-Alkoxy, oder $C_1$-$C_4$-Alkyl bedeutet; $R_2$ für Wasserstoff, $C_1$-$C_4$-Alkyl oder Hydroxy-$C_2$-$C_4$-Alkyl steht, worin Hydroxy vom Stickstoffatom durch mindestens 2 Kohlenstoffatome getrennt ist; $R_3$ Wasserstoff, $C_1$-$C_4$-Alkyl, $C_1$-$C_4$-Alkoxy, $C_1$-$C_4$-Alkylthio,

Halogen oder Trifluormethyl bedeutet; $C_nH_{2n}$ für Äthylen oder Propylen steht; ihre N-Oxide und Salze herstellt.

5. Verfahren gemäss Anspruch 1, dadurch gekennzeichnet, dass man Verbindungen der Formel II, worin $R_1$ Wasserstoff, Halogen, $C_1$-$C_4$-Alkylthio, $C_1$-$C_4$-Alkoxy oder $C_1$-$C_4$-Alkyl; $R_2$ Wasserstoff, $C_1$-$C_4$Alkyl oder Hydroxy-$C_2$-$C_4$-alkyl bedeuten, worin Hydroxy vom Stickstoffatom durch 2 oder 3 Kohlenstoffatome getrennt ist; $R_3$ für Wasserstoff, $C_1$-$C_4$-Alkyl, Halogen oder Trifluormethyl steht; $C_nH_{2n}$ Äthylen bedeutet, ihre N-Oxide und therapeutisch verwendbare Salze davon herstellt.

6. Verfahren gemäss Anspruch 1, dadurch gekennzeichnet, dass man Verbindungen der Formel II, worin $R_1$ Wasserstoff, Methyl, Äthyl, Chlor, Methylthio oder Methoxy; $R_2$ Wasserstoff, Methyl, Äthyl, Propyl, 2-Hydroxyäthyl oder 3-Hydroxypropyl; $R_3$ Wasserstoff, Methyl, Methoxy, Fluor, Chlor oder Trifluormethyl bedeutet; und $C_nH_{2n}$ Äthylen bedeutet, und therapeutisch verwendbare salze herstellt.

7. Verfahren gemäss Anspruch 1, dadurch gekennzeichnet, dass man 2-Methyl-5-(4-methyl-1-piperazinyl)-11H-1,2,4-triazol[2,3-c]-[1,3]benzodiazepin und seine therapeutisch verwendbaren Salze herstellt.

8. Verfahren zur Herstellung von pharmazeutischen Präparaten, dadurch gekennzeichnet, dass man Verbindungen der Formel I gemäss Anspruch 1, ihre N-Oxide und Salze, zusammen mit einem pharmazeutischen Trägermaterial vermischt.

**Claims** for the Contracting States: BE, CH, DE, FR, GB, IT, LI, LU, NL, SE

1. Compounds of the formula

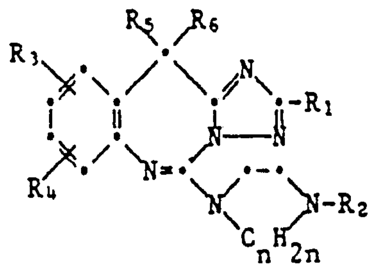

(I),

wherein $R_1$ is hydrogen, $C_1$-$C_4$alkylthio, amino, ($C_1$-$C_4$alkanoyl-, $C_1$-$C_4$alkoxycarbonyl-, carbamoyl-, sulfamoyl-, $C_1$-$C_4$monoalkyl-, or di-$C_1$-$C_4$alkyl-carbamoyl-or -sulfamoyl-, halosulfonyl-, phenyl-$C_1$-$C_2$alkoxy-carbonyl-, $C_1$-$C_4$alkyl- or di-$C_1$-$C_4$alkyl-)amino, $C_1$-$C_4$alkoxy, ($C_1$-$C_4$alkanoyl-, $C_1$-$C_4$alkoxycarbonyl-, carbamoyl-, sulfamoyl-, mono-$C_1$-$C_4$alkyl- or di-$C_1$-$C_4$alkyl-carbamoyl- or -sulfamoyl-, halosulfonyl- or phenyl-$C_1$-$C_2$alkoxycarbonyl-)oxy, $C_1$-$C_4$alkyl, acetyl, propionyl, hydroxy, halogen, trifluoromethyl, cyano, carboxy, methoxy- or ethoxy-carbonyl, carbamoyl, mono- or di-methyl- or -ethyl-carbamoyl, hydroxy-$C_1$-$C_4$alkyl or di-$C_1$-$C_4$alkylamino-$C_1$-$C_4$alkyl;

$C_nH_{2n}$ is $C_2$-$C_4$alkylene separating the two nitrogen atoms by 2 or 3 carbon atoms;

$R_2$ is hydrogen, $C_1$-$C_7$alkyl, allyl, propargyl, acetyl, propionyl, phenyl-$C_1$-$C_3$alkyl which can be substituted at the phenyl ring by halogen, $C_1$-$C_4$alkoxy or by $C_1$-$C_4$alkyl, methoxy- or ethoxy-carbonyl, phenylmethoxycarbonyl, phenylethoxycarbonyl, 2-hydroxy-(ethyl or propyl), 3-hydroxy-(propyl or butyl), 4-hydroxybutyl, $C_2$-$C_4$alkanoyl-oxy-$C_2$-$C_4$alkyl, phenoxy-$C_2$-$C_4$alkyl which can be substituted at the phenyl ring by halogen, $C_1$-$C_4$alkoxy or by $C_1$-$C_4$alkyl, or $C_1$-$C_4$alkoxy-$C_2$-$C_4$alkyl;

$R_3$ and $R_4$ independently represent hydrogen, $C_1$-$C_7$alkyl, $C_1$-$C_4$alkoxy, $C_1$-$C_4$alkylthio, halogen, trifluoromethyl, hydroxy, acetoxy, propionyloxy, sulfamoyl, mono- or di-$C_1$-$C_4$alkylsulfamoyl, and $R_5$ and $R_6$ represent hydrogen or $C_1$-$C_4$alkyl; and N-oxides and salts thereof.

2. Compounds according to claim 1 of the formula I wherein $R_1$ is hydrogen, $C_1$-$C_4$alkyl, halogen, trifluoromethyl, $C_1$-$C_4$alkoxy or $C_1$-$C_4$alkylthio; $C_nH_{2n}$ is $C_2$-$C_4$alkylene separating the two nitrogen atoms by 2 or 3 carbon atoms; $R_2$ is hydrogen, $C_1$-$C_4$alkyl, methoxycarbonyl, ethoxycarbonyl or hydroxy-$C_2$-$C_4$alkyl; $R_3$ represents hydrogen, $C_1$-$C_4$alkyl, $C_1$-$C_4$alkoxy, $C_1$-$C_4$alkyl-thio, halogen or trifluoromethyl; $R_4$ represents hydrogen; and $R_5$ and $R_6$ represent hydrogen or $C_1$-$C_4$alkyl; and N-oxides and salts thereof.

3. Compounds according to claim 1 of the formula I wherein $R_1$ is hydrogen, methyl, ethyl, methylthio, chloro, methoxy or trifluoromethyl; n represents the integer 2 or 3; $R_2$ is hydrogen, $C_1$-$C_3$alkyl, methoxycarbonyl, ethoxycarbonyl, hydroxyethyl or hydroxypropyl; $R_3$ represents hydrogen, methyl, methoxy, methylthio, chloro or trifluoromethyl; $R_4$ represents hydrogen; and $R_5$ and $R_6$ represent hydrogen or methyl; and N-oxides and salts thereof.

4. Compounds according to claim 1 having the formula II

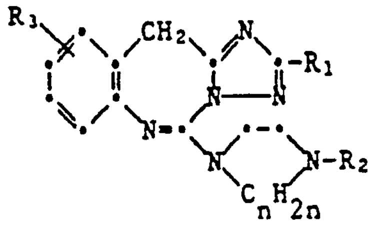

(II),

wherein $R_1$ represents hydrogen, halogen, trifluoromethyl, $C_1$-$C_4$alkylthio, $C_1$-$C_4$alkoxy or $C_1$-$C_4$alkyl; $R_2$ represents hydrogen, $C_1$-$C_4$alkyl or hydroxy-$C_2$-$C_4$alkyl wherein the hydroxy group is separated from the nitrogen atom by at least 2 carbon atoms; $R_3$ represents hydrogen, $C_1$-$C_4$alkyl, $C_1$-$C_4$alkoxy, $C_1$-$C_4$alkylthio, halogen or trifluoromethyl; $C_nH_{2n}$ represents ethylene or propylene; and N-oxides and salts thereof.

5. Compounds according to claim 1 of the formula II wherein $R_1$ represents hydrogen, halogen, $C_1$-$C_4$alkylthio, $C_1$-$C_4$alkoxy or $C_1$-$C_4$alkyl; $R_2$ represents hydrogen, $C_1$-$C_4$alkyl or hydroxy-$C_2$-$C_4$alkyl wherein the hydroxy group is separated from the nitrogen atom by 2 or 3 carbon atoms; $R_3$ represents hydrogen, $C_1$-$C_4$alkyl, halogen or trifluoromethyl; $C_nH_{2n}$ represents ethylene; and N-oxides and therapeutically acceptable salts thereof.

6. Compounds according to claim 1 of the formula II wherein $R_1$ represents hydrogen, methyl, ethyl, chloro, methylthio or methoxy; $R_2$ represents hydrogen, methyl, ethyl, propyl, 2-hydroxyethyl or 3-hydroxypropyl; $R_3$ is hydrogen, methyl, methoxy, fluoro, chloro or trifluoromethyl; and $C_nH_{2n}$ represents ethylene; and therapeutically acceptable salts.

7. 2-methyl-5-(4-methyl-1-piperazinyl)-11H-1,2,4-triazolo[2,3-c]-[1,3]benzodiazepine and therapeutically acceptable salts thereof.

8. Pharmaceutical preparations containing compounds of the formula I according to claim 1, their N-oxides and salts, together with a pharmaceutical carrier.

9. The compounds mentioned in claim 1 for use in a method for the therapeutic treatment of the human or animal body.

10. The compounds mentioned in claim 1 as neuroleptic agents.

11. The compounds mentioned in claim 1 as antihistaminic agents.

12. The use of the compounds mentioned in claim 1 for the manufacture of pharmaceutical preparations.

13. A process for the manufacture of compounds of the formula I according to claim 1, characterized in that

a) a compound of the formula III

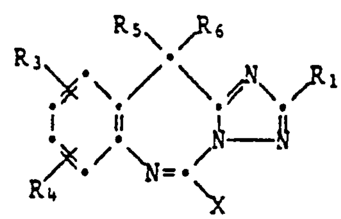

(III),

wherein X is a group detachable together with hydrogen or with an alkali metal, and the other symbols are as defined under the formula I, is condensed with a compound of the formula IV

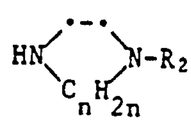

(IV)

or with an alkali metal derivative thereof wherein $R_2$ is as defined under the formula I, or

b) a compound of the formula VI

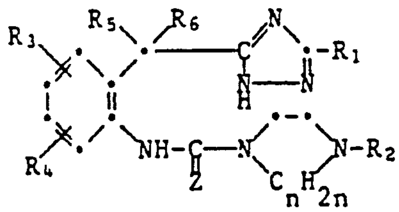

(VI),

wherein Z is oxygen, sulfur or NH, and the other symbols are as defined above, is cyclized under dehydrating, dehydrosulfurating or deaminating conditions, and, if desired, a resulting free compound is converted into a salt or a resulting salt is converted into the free compound or into another salt, and/or, if desired, a resulting mixture of isomers or racemates is resolved into the single isomers or racemates, and/or, if desired, resulting racemates are resolved into the optical antipodes.

**Claims** for the Contracting State: AT

1. A process for the manufacture of compounds of the formula

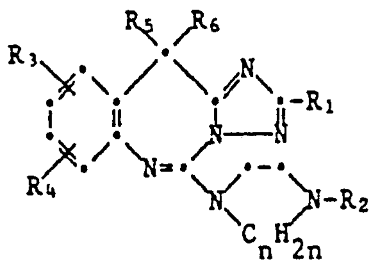

(I),

wherein $R_1$ is hydrogen, $C_1$-$C_4$alkylthio, amino, ($C_1$-$C_4$alkanoyl-, $C_1$-$C_4$alkoxycarbonyl-, carbamoyl-, sulfamoyl-, $C_1$-$C_4$monoalkyl-, or di-$C_1$-$C_4$alkyl-carbamoyl- or -sulfamoyl-, halosulfonyl-, phenyl-$C_1$-$C_2$alkoxy-carbonyl-, $C_1$-$C_4$alkyl- or di-$C_1$-$C_4$alkyl-)amino, $C_1$-$C_4$alkoxy, ($C_1$-$C_4$alkanoyl-, $C_1$-$C_4$alkoxycarbonyl-, carbamoyl-, sulfamoyl-, mono-$C_1$-$C_4$alkyl- or di- $C_1$-$C_4$alkyl-carbamoyl-or -sulfamoyl-, halosulfonyl- or phenyl-$C_1$-$C_2$alkoxycarbonyl-)oxy, acyloxy, $C_1$-$C_4$alkyl, acetyl, propionyl, hydroxy, halogen, trifluoromethyl, cyano, carboxy, methoxy- or ethoxy-carbonyl, carbamoyl, mono- or di-methyl- or -ethyl-carbamoyl, hydroxy- $C_1$-$C_4$alkyl or di-$C_1$-$C_4$alkylamino-$C_1$-$C_4$alkyl;

$C_nH_{2n}$ is $C_2$-$C_4$alkylene separating the two nitrogen atoms by 2 or 3 carbon atoms;

$R_2$ is hydrogen, $C_1$-$C_7$alkyl, allyl, propargyl, acetyl, propionyl, phenyl-$C_1$-$C_3$alkyl which can be substituted at the phenyl ring by halogen, $C_1$-$C_4$alkoxy or by $C_1$-$C_4$alkyl, methoxy- or ethoxy-carbonyl, phenylmethoxycarbonyl, phenylethoxycarbonyl, 2-hydroxy-(ethyl or propyl), 3-hydroxy-(propyl or butyl), 4-hydroxybutyl, $C_2$-$C_4$alkanoyloxy-$C_2$-$C_4$alkyl, phenoxy-$C_2$-$C_4$alkyl which can be substituted at the phenyl ring by halogen, $C_1$-$C_4$alkoxy or by $C_1$-$C_4$alkyl, or $C_1$-$C_4$alkoxy-$C_2$-$C_4$alkyl;

$R_3$ and $R_4$ independently represent hydrogen, $C_1$-$C_7$alkyl, $C_1$-$C_4$alkoxy, $C_1$-$C_4$alkylthio, halogen, trifluoromethyl, hydroxy, acetoxy, propionyloxy, sulfamoyl, mono- or di- $C_1$-$C_4$alkylsulfamoyl, and $R_5$ and $R_6$ represent hydrogen or $C_1$-$C_4$alkyl; and N-oxides and salts thereof, characterized in that

a) a compound of the formula III

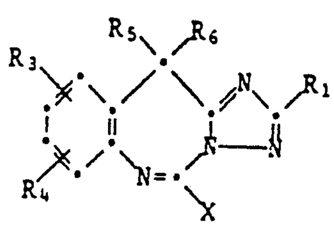

(III),

wherein X is a group detachable together with hydrogen or with an alkali metal, and the other symbols are as defined under the formula I, is condensed with a compound of formula IV

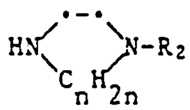

(IV)

or with an alkali metal derivative thereof in which $R_2$ is as defined under the formula I, or

b) a compound of the formula VI

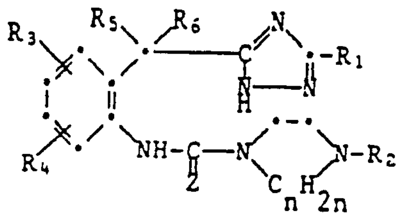

(VI),

wherein Z is oxygen, sulfur or NH, and the other symbols are as defined above, is cyclized under dehydrating, dehydrosulfurating or deaminating conditions, and, if desired, a resulting free compound is converted into a salt or a resulting salt is converted into the free compound or into another salt, and/or, if desired, a resulting mixture of isomers or racemates is resolved into the single isomers or racemates, and/or, if desired, resulting racemates are resolved into the optical antipodes.

2. A process according to claim 1, characterized in that compounds of the formula I wherein $R_1$ is hydrogen, $C_1$-$C_4$alkyl, halogen, trifluoromethyl, $C_1$-$C_4$alkoxy or $C_1$-$C_4$alkylthio; $C_nH_{2n}$ is $C_2$-$C_4$alkylene separating the two nitrogen atoms by 2 or 3 carbon atoms; $R_2$ is hydrogen, $C_1$-$C_4$alkyl, methoxycarbonyl, ethoxycarbonyl or hydroxy-$C_2$-$C_4$alkyl; $R_3$ represents hydrogen, $C_1$-$C_4$alkyl, $C_1$-$C_4$-alkoxy, $C_1$-$C_4$alkylthio, halogen or trifluoromethyl; $R_4$ represents hydrogen; and $R_5$ and $R_6$ represent hydrogen or $C_1$-$C_4$alkyl; and N-oxides and salts thereof, are manufactured.

3. A process according to claim 1, characterized in that compounds of the formula I wherein $R_1$ is hydrogen, methyl, ethyl, methylthio, chloro, methoxy or trifluoromethyl; n represents the integer 2 or 3; $R_2$ is hydrogen, $C_1$-$C_3$alkyl, methoxycarbonyl, ethoxycarbonyl, hydroxyethyl or hydroxypropyl; $R_3$ represents hydrogen, methyl, methoxy, methylthio, chloro or trifluoromethyl; $R_4$ represents hydrogen; and $R_5$ and $R_6$ represent hydrogen or methyl; and N-oxides and salts thereof, are manufactured.

4. A process according to claim 1, characterized in that compounds having the formula II

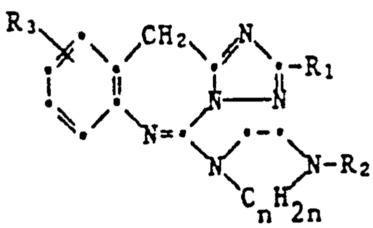

(II),

wherein $R_1$ represents hydrogen, halogen, trifluoromethyl, $C_1$-$C_4$alkylthio, $C_1$-C4alkoxy or $C_1$-$C_4$alkyl; $R_2$ represents hydrogen, $C_1$-$C_4$alkyl or hydroxy-$C_2$-$C_4$alkyl wherein the hydroxy group is separated from the nitrogen atom by at least 2 carbon atoms; $R_3$ represents hydrogen, $C_1$-$C_4$-alkyl, $C_1$-$C_4$alkoxy, $C_1$-$C_4$alkylthio, halogen or trifluoromethyl; $C_nH_{2n}$ represents ethylene or propylene; and N-oxides and salts thereof, are manufactured.

5. A process according to claim 1, characterized in that compounds of the formula II wherein $R_1$ represents hydrogen, halogen, $C_1$-$C_4$alkylthio, $C_1$-$C_4$alkoxy or $C_1$-$C_4$alkyl; $R_2$ represents hydrogen, $C_1$-$C_4$alkyl or hydroxy-$C_2$-$C_4$alkyl wherein the hydroxy group is separated from the nitrogen atom by 2 or 3 carbon atoms; $R_3$ represents hydrogen, $C_1$-$C_4$alkyl, halogen or trifluoromethyl; $C_nH_{2n}$ represents ethylene, and N-oxides and therapeutically acceptable salts thereof, are manufactured.

6. A process according to claim 1, characterized in that compounds of the formula II wherein $R_1$ represents hydrogen, methyl, ethyl, chloro, methylthio or methoxy;

$R_2$ represents hydrogen, methyl, ethyl, propyl, 2-hydroxy-ethyl or 3-hydroxypropyl; $R_3$ is hydrogen, methyl, methoxy, fluoro, chloro or trifluoromethyl; and $C_nH_{2n}$ represents ethylene, and therapeutically acceptable salts, are manufactured.

7. A process according to claim 1, characterized in that 2-methyl-5-(4-methyl-1-piperazinyl)-11H-1,2,4-triazolo-[2,3-c][1,3]benzodiazepine, and therapeutically acceptable salts thereof, are manufactured

8. A process for the manufacture of pharmaceutical preparations, characterized in that compounds of the formula I according to claim 1, N-oxides and salts thereof, are mixed together with a pharmaceutical carrier.

**Revendications** pour les Etats contractants: BE, CH, DE, FR, GB, IT, LI, LU, NL, SE

1. Composés de formule

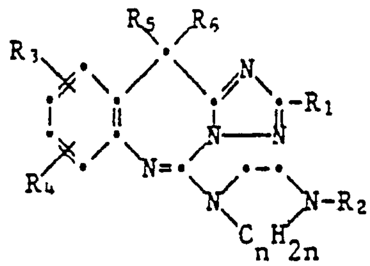

(I),

où R représente l'hydrogène, un alkyle en $C_1$-$C_4$ thio, un amino, un (alcanoyle en $C_1$-$C_4$, alcoxy en $C_1$ -$C_4$ carbonyl-, carbamoyl-, sulfamoyl-, monoalkyle en $C_1$-$C_4$ ou dialkyle en $C_1$-$C_4$ carbamoyl- ou sulfamoyl-, halosulfonyl-, phénylalcoxy en $C_1$-$C_2$ carbonyl-, alkyle en $C_1$-$C_4$ ou dialkyle en $C_1$-$C_4$)amino, un alcoxy en $C_1$-$C_4$, un (alcanoyle en $C_1$-$C_4$, alcoxy en $C_1$-$C_4$ carbonyl-, carbamoyl-, sulfamoyl-, monoalkyle en $C_1$-$C_4$ ou dialkyle en $C_1$-$C_4$ carbamoyl- ou sulfamoyl-, halosulfonyl- ou phénylalcoxy en $C_1$-$C_2$ carbonyl-), oxy, un alkyle en $C_1$-$C_4$, l'acétyle, le propionyle, l'hydroxy, un halogène, le trifluorométhyle, un cyano, un carboxy, un méthoxy- ou éthoxycarbonyle, le carbamoyl, le mono- ou diméthyl- ou éthylcarbamoyl, un hydroxyalkyle en $C_1$-$C_4$ ou un dialkyle en $C_1$-$C_4$ aminoalkyle en $C_1$-$C_4$;

$C_n$-$H_{2n}$ représente un alkylène en $C_2$-$C_4$, séparant les deux atomes d'azote par 2 ou 3 atomes de carbone;

$R_2$ représente l'hydrogène, un alkyle en $C_1$-$C_7$, l'allyle, le propargyle, l'acétyle, le propionyle, un phénylalkyle en $C_1$-$C_3$, pouvant être substitué sur le noyau phényle par un halogène, un alcoxy en $C_1$-$C_4$ ou un alkyle en $C_1$-$C_4$, le méthoxy- ou l'éthoxy-carbonyle, le phénylméthoxycarbonyle, le phényléthoxycarbonyle, le 2-hydroxy-(éthyle ou propyle), le 3-hydroxy-(propyle ou butyle), le 4-hydroxybutyle, un alcanoyloxy en $C_2$-$C_4$ alkyle en $C_2$-$C_4$, un phénoxyalkyle en $C_2$-$C_4$, pouvant être substitué sur le noyau phényle par un halogène, par un alcoxy en $C_1$-$C_4$ ou par un alkyle en $C_1$-$C_4$, ou un alcoxy en $C_1$-$C_4$ alkyle en $C_2$-$C_4$;

$R_3$ et $R_4$ représentent indépendamment l'un de l'autre, l'hydrogène, un alkyle en $C_1$-$C_7$, un alcoxy en $C_1$-$C_4$, un alkyle en $C_1$-$C_4$ thio, un halogène, le trifluorométhyle, l'hydroxy, l'acétoxy, le propionyloxy, le sulfamoyl, un mono- ou dialkyle en $C_1$-$C_4$ sulfamoyl et

$R_5$, et $R_6$ représentent l'hydrogène ou un alkyle en $C_1$-$C_4$; leurs N-oxydes et leurs sels.

2. Composés de formule I selon la revendication 1, où $R_1$ représente l'hydrogène, un alkyl en $C_1$-$C_4$, un halogène, le trifluorométhyle, un alcoxy en $C_1$-$C_4$ ou un alkyle en $C_1$-$C_4$ thio; $C_nH_{2n}$ représente un alkylène en $C_2$-$C_4$, séparant les deux atomes d'azote par 2 ou 3 atomes de C; $R_2$ représente l'hydrogène, un alkyle en $C_1$-$C_4$, le méthoxycarbonyle, l'éthoxycarbonyle ou un hydroxyalkyle en $C_2$-$C_4$; $R_3$ représente l'hydrogène, un alkyle en $C_1$-$C_4$, un alcoxy en $C_1$-$C_4$, un alkyle en $C_1$-$C_4$ thio, un halogène ou le trifluorométhyle; $R_4$ représente l'hydrogène et $R_5$ et $R_6$ représentent l'hydrogène ou un alkyle en $C_1$-$C_4$; leurs N-oxydes et leurs sels.

3. Composés de formule I selon la revendication 1, où $R_1$ représente l'hydrogène, le méthyle, l'éthyle, le méthylthio, le chlore, le méthoxy ou le trifluorométhyle, n est un nombre entier égal à 2 ou 3;

$R_2$ représente l'hydrogène, un alkyle en $C_1$-$C_3$, le méthoxycarbonyle, l'éthoxycarbonyle, l'hydroxyéthyle ou l'hydroxypropyle; $R_3$ représente l'hydrogène, le méthyle, le méthoxy, le méthylthio, le chlore ou le trifluorométhyle;

$R_4$ représente l'hydrogène et $R_5$ et $R_6$ représentent l'hydrogène ou le méthyle; leurs N-oxydes et leurs sels.

4. Composés de formule II selon la revendication 1,

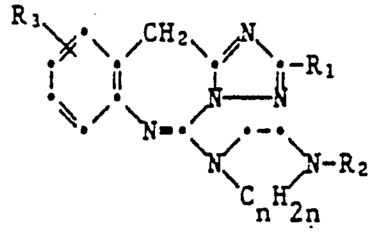

(II),

où $R_1$ représente l'hydrogène, un halogène, le trifluorométhyle, un alkyle en $C_1$-$C_4$ mercapto, un alcoxy en $C_1$-$C_4$ ou un alkyle en $C_1$-$C_4$; $R_2$ représente l'hydrogène, un alkyle en $C_1$-$C_4$ ou un hydroxyalkyle en $C_2$-$C_4$, l'hydroxy étant séparé de l'atome d'azote par au moins 2 atomes de carbone; $R_3$ représente l'hydrogène, un alkyle en $C_1$-$C_4$, un alcoxy en $C_1$-$C_4$, un alkyle en $C_1$-$C_4$ thio, un halogène ou le trifluorométhyle; $C_nH_{2n}$ représente l'éthylène ou le propylène; leurs N-oxydes et leurs sels.

5. Composés de formule II selon la revendication 1, où $R_1$ représente l'hydrogène, un halogène, un alkyle en

$C_1$-$C_4$ thio, un alcoxy en $C_1$-$C_4$ ou un alkyle en $C_1$-$C_4$; $R_2$ représente l'hydrogéne, un alkyle en $C_1$-$C_4$ ou un hydroxyalkyle en $C_2$-$C_4$, l'hydroxy étant séparé de l'atome d'azote par 2 ou 3 atomes de carbone; $R_3$ représente l'hydrogène, un alkyle en $C_1$-$C_4$, un halogène ou le trifluorométhyle; $C_nH_{2n}$ représente l'éthylène; leurs N-oxydes et leurs sels thérapeutiquement acceptables.

6. Composés de formule II selon la revendication 1, où $R_1$ représente l'hydrogène, le méthyle, l'éthyle, le chlore, le méthylthio ou le méthoxy; $R_2$ représente l'hydrogène, le méthyle, l'éthyle, le propyle, le 2-hydroxyéthyle ou le 3-hydroxypropyle; $R_3$ représente l'hydrogène, le méthyle, le méthoxy, le fluor, le chlore ou le trifluorométhyle et $C_nH_{2n}$ représente l'éthylène et les sels pharmaceutiquement acceptables.

7. La 2-méthyl-5-(4-méthyl-1-pipérazinyl)-11H-1,2,4-triazol[2,3-c][1,3]benzodiazépine et ses sels thérapeutiquement acceptables.

8. Préparations pharmaceutiques contenant des composés de formule I selon la revendication 1, leurs N-oxydes et leurs sels, ensemble avec un support pharmaceutique.

9. Composés définis dans la revendication 1 utilisés dans un procédé de traitement thérapeutique du corps humain ou animal.

10. Composés définis dans la revendication 1 comme agents neuroleptiques.

11. Composés définis dans la revendication 1 comme agents antihistaminiques.

12. Utilisation des composés définis dans la revendication 1 pour la préparation on de compositions pharmaceutiques.

13. Procédé de préparation de composés de formule I selon la revendication 1, caractérisé

a) en ce que l'on condense un composé de formule III

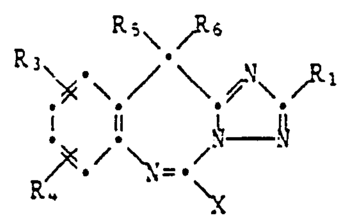

(III),

où X représente un groupe clivable ensemble avec l'hydrogène ou un métal alcalin, et où les autres symboles ont la signification donnée à propos de la formule I, avec un composé de formule IV

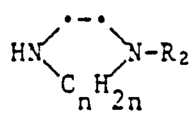

(IV)

ou avec un dérivé de métal alcalin de celui-ci, dans laquelle $R_2$ a la signification donnée à propos de la formule I ou

b) en ce que l'on cyclise un composé de formule VI

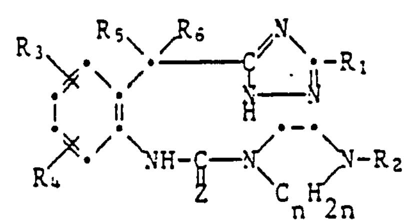

(VI),

où Z représente un atome d'oxygène ou de soufre ou NH et où les autres symboles ont la signification donnée ci-dessus, dans des conditions de déshydratation, de déshydrosulfuration ou de désamination et, si désiré, en ce que l'on transforme le composé libre obtenu en un sel ou le sel obtenu en un composé libre ou en un autre sel, et/ou, si désiré, en ce que l'on sépare individuellement les isomères ou racémates du mélange d'isomères ou de racémates obtenu, et/ou, si désiré, en ce que l'on sépare les racémates obtenus en antipodes optiques.

**Revendications** pour l'Etat Contractant: AT

1. Procédé de préparation de composés de formule

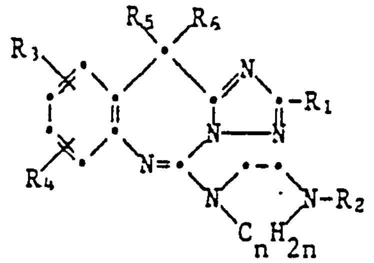

(I),

où R représente l'hydrogène, un alkyle en $C_1$-$C_4$ thio, un amino, un (alcanoyle en $C_1$-$C_4$, alcoxy en $C_1$-$C_4$ carbonyl-, carbamoyl-, sulfamoyl, monoalkyle en $C_1$-$C_4$ ou dialkyle en $C_1$-$C_4$ carbamoyl- ou sulfamoyl-, halosulfonyl-, phénylalcoxy en $C_1$-$C_2$ carbonyl-, alkyle en $C_1$-$C_4$ ou dialkyle en $C_1$-$C_4$) amino, un alcoxy en $C_1$-$C_4$, un (alcanoyle en $C_1$ -$C_4$, alcoxy en $C_1$-$C_4$ carbonyl-, carbamoyl-, sulfamoyl-, monoalkyle en $C_1$-$C_4$ ou dialkyle en $C_1$-$C_4$ carbamoyl- ou sulfamoyl-, monoalkyle en $C_1$-$C_4$ ou dialkyle en $C_1$-$C_4$ carbamoyl- ou sulfamoyl-, halosulfonyl- ou phénylalcoxy en $C_1$-$C_2$carbonyl-)oxy, un alkyle en $C_1$-$C_4$, l'acétyle, le propionyle, l'hydroxy, un halogène, le trifluorométhyle, un cyano, un carboxy, un méthoxy- ou éthoxycarbonyle, le carbamoyl, le mono- ou diméthyl- ou éthylcarbamoyl, un hydroxyalkyle en $C_1$-$C_4$ ou un dialkyle en $C_1$-$C_4$ aminoalkyle en $C_1$-$C_4$;

$C_nH_{2n}$ représente un alkylène en $C_2$-$C_4$, séparant les deux atomes d'azote par 2 ou 3 atomes de carbone;

$R_2$ représente l'hydrogène, un alkyle en $C_1$-$C_7$, l'allyle, le propargyle, l'acétyle, le propionyle, un phénylalkyle en $C_1$-$R_3$, pouvant être substitué sur le noyau phényle par un halogène, un alcoxy en $C_1$-$C_4$ ou un alkyle en $C_1$-$C_4$, le méthoxy- ou l'éthoxy-carbonyle, le phénylméthoxycarbonyle, le phényléthoxycarbonyle, le 2-hydroxy(éthyle ou propyle), le 3-hydroxy-(propyle ou butyle), le 4-hydroxybutyle, un alcanoyloxy en $C_2$-$C_4$ alkyle en $C_2$-$C_4$, un phénoxyalkyle en $C_2$-$C_4$, pouvant être substitué sur le noyau phényle par un halogène, par un alcoxy en $C_1$-$C_4$ ou par un alkyle en $C_1$-$C_4$, ou un alcoxy en $C_1$-$C_4$ alkyle en $C_2$-$C_4$;

$R_3$ et $R_4$ représentent indépendamment l'un de l'autre, l'hydrogène, un alkyle en $C_1$-$C_7$, un alcoxy en $C_1$-$C_4$, un alkyle en $C_1$-$C_4$ thio, un halogène, le trifluorométhyle, l'hydroxy, l'acétoxy, le propionyloxy, le sulfamoyl, un mono- ou dialkyle en $C_1$-$C_4$ sulfamoyl et

$R_5$ et $R_6$ représentent l'hydrogène ou un alkyle en $C_1$-$C_4$; leurs N-oxydes et leurs sels.

a) en ce que l'on condense un composé de formule III

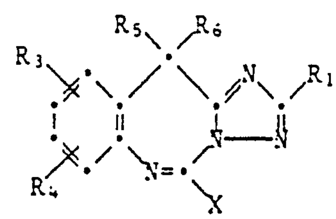

(III),

où X représente un groupe clivable ensemble avec l'hydrogène ou un métal alcalin, et où les autres symboles ont la signification donnée à propos de la formule I, avec un composé de formule IV

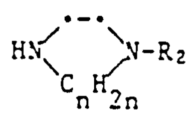

(IV)

ou avec un dérivé de métal alcalin de celui-ci, dans laquelle $R_2$ a la signification donnée à propos de la formule I ou

b) en ce que l'on cyclise un composé de formule VI

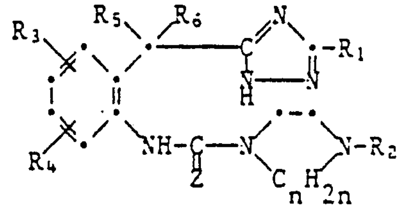

(VI),

où Z représente un atome d'oxygène ou de soufre ou NH et où les autres symboles ont la signification donnée ci-dessus, dans des conditions de déshydratation, de déshydrosulfuration ou de désamination et, si désiré, en

ce que l'on transforme le composé libre obtenu en un sel ou le sel obtenu en un composé libre ou en un autre sel, et/ou, si désiré, en ce que l'on sépare individuellement les isomères ou racémates du mélange d'isomères ou de racémates obtenus, et/ou, si désiré, en ce que l'on sépare les racémates obtenus en antipodes optiques.

2. Procédé selon la revendication 1, caractérisé en ce que l'on prépare des composés de formule I où $R_1$ représente l'hydrogène, un alkyle en $C_1$-$C_4$, un halogène, le trifluorométhyle, un alcoxy en $C_1$-$C_4$ ou un alkyle en $C_1$-$C_4$ thio; $C_nC_{2n}$ représente un alkylène en $C_2$-$C_4$, séparant les deux atomes d'azote par 2 ou 3 atomes de C; $R_2$ représente l'hydrogène, un alkyle en $C_1$-$C_4$, le méthoxycarbonyle, l'éthoxycarbonyle ou un hydroxyalkyle en $C_2$-$C_4$; $R_3$ représente l'hydrogène, un alkyle en $C_1$-$C_4$, un alcoxy en $C_1$-$C_4$, un alkyle en $C_1$-$C_4$ thio, un halogène ou le trifluorométhyle; $R_4$ représente l'hydrogène et $R_5$ et $R_6$ représentent l'hydrogène ou un alkyle en $C_1$-$C_4$; leurs N-oxydes et leurs sels.

3. Procédé selon la revendication 1, caractérisé en ce que l'on prépare des composés de formule I où $R_1$ représente l'hydrogène, le méthyle, l'éthyle, le méthylthio, le chlore, le méthoxy ou le trifliuorométhyle, n est un nombre entier égal à 2 ou 3; $R_2$ représente l'hydrogène, un alkyle en $C_1$-$C_3$, le méthoxycarbonyle, l'éthoxycarbonyle, l'hydroxyéthyle ou l'hydroxypropyle;

$R_3$, représente l'hydrogène, le méthyle, le méthoxy, le méthylthio, le chlore ou le trifluorométhyle; $R_4$ représente l'hydrogène et $R_5$ et $R_6$ représentent l'hydrogène ou le méthyle; leurs N-oxydes et leurs sels.

4. Procédé selon la revendication 1, caractérisé en ce que l'on prépare des composés de formule II

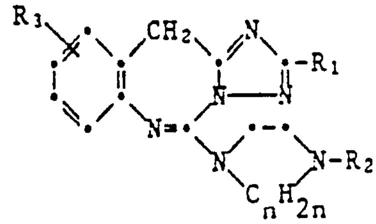

(II),

où $R_1$ représente l'hydrogène, un halogène, le trifluorométhyle, un alkyle en $C_1$-$C_4$ mercapto, un alcoxy en $C_1$-$C_4$ ou un alkyle en $C_1$-$C_4$; $R_2$ représente l'hydrogène, un alkyle en $C_1$-$C_4$ ou un hydroxyalkyle en $C_2$-$C_4$, l'hydroxy étant séparé de l'atome d'azote par au moins 2 atomes de carbone; $R_3$ représente l'hydrogène, un alkyle en $C_1$-$C_4$, un alcoxy en $C_1$-$C_4$, un alkyle en $C_1$-$C_4$ thio, un halogène ou le trifluorométhyle; $C_nH_{2n}$ représente l'éthylène ou le propylène; leurs N-oxydes et leurs sels.

5. Procédé selon la revendication 1, caractérisé en ce que l'on prépare des composés de formule II où $R_1$ représente l'hydrogène, un halogène, un alkyle en $C_1$-$C_4$ thio, un alcoxy en $C_1$-$C_4$ ou un alkyle en $C_1$-$C_4$;

$R_2$ représente l'hydrogène, un alkyle en $C_1$-$C_4$ ou un hydroxyalkyle en $C_2$-$C_4$, l'hydroxy étant séparé de l'atome d'azote par 2 ou 3 atomes de carbone; $R_3$ représente l'hydrogène, un alkyle en $C_1$-$C_4$, un halogène ou le trifluorométhyle; $C_nH_{2n}$ représente l'éthylène; leurs N-oxydes et leurs sels thérapeutiquement acceptables.

6. Procédé selon la revendication 1, caractérisé en ce que l'on prépare des composés de formule II où $R_1$ représente l'hydrogène, le méthyle, l'éthyle, le chlore, le méthylthio ou le méthoxy; $R_2$ représente l'hydrogène, le méthyle, l'éthyle, le propyle, le 2-hydroxyéthyle ou le 3-hydroxypropyle; $R_3$ représente l'hydrogène, le méthyle, le méthoxy, le fluor, le chlore ou le trifluorométhyle et $C_nH_{2n}$ représente l'éthylène et les sels pharmaceutiquement acceptables.

7. Procédé selon la revendication 1, caractérisé en ce que l'on prépare la 2-méthyl-5-(4-méthyl-1-pipérazinyl)-11H-1,2,4-triazol[2,3-c][1,3]benzodizépine et ses sels thérapeutiquement acceptables.

8. Procédé de préparation de compositions pharmaceutiques, caractérisé en ce que l'on mélange des composés de formule I selon la revendication 1, leurs N-oxydes et leurs sels, ensemble avec un support pharmaceutique.